# EUROPÄISCHE PATENTSCHRIFT

(11) **EP 0 741 695 B1**
(45) Veröffentlichungstag und Bekanntmachung des Hinweises auf die Patenterteilung: **17.11.1999**
(21) Anmeldenummer: 95906953.5
(22) Anmeldetag: 16.01.1995
(51) Int. Cl.: C07C 259/06, C07D 417/04, C07D 285/08, C07D 213/64, C07D 333/32, C07D 213/53, C07D 239/52, C07D 309/12, C07D 277/32, A01N 37/36, A01N 37/44

(54) **HYDROXAMSÄUREDERIVATE, VERFAHREN ZU IHRER HERSTELLUNG UND VERWENDUNG ALS FUNGIZIDE**
HYDROXAMIC-ACID DERIVATIVES, METHOD OF PREPARING THEM AND THEIR USE AS FUNGICIDES
DERIVES DE L'ACIDE HYDROXAMIQUE, LEUR PROCEDE DE FABRICATION ET LEUR UTILISATION COMME FONGICIDES

(30) Priorität: 28.01.1994 DE 4402533
(43) Veröffentlichungstag der Anmeldung: 13.11.1996
(73) Patentinhaber: BAYER AG, 51368 Leverkusen (DE)
(72) Erfinder: KRÜGER, Bernd-Wieland, D-51467 Bergisch Gladbach (DE); ASSMANN, Lutz, D-23701 Eutin (DE); GAYER, Herbert, D-40789 Monheim (DE); GERDES, Peter, D-52080 Aachen (DE); HEINEMANN, Ulrich, D-42799 Leichlingen (DE); KUHNT, Dietmar, D-51373 Leverkusen (DE); SEITZ, Thomas, D-40764 Langenfeld (DE); GALLENKAMP, Bernd, D-42113 Wuppertal (DE); TIEMANN, Ralf, D-51375 Leverkusen (DE); HÄNSSLER, Gerd, D-51381 Leverkusen (DE); DEHNE, Heinz-Wilhelm, D-53125 Bonn (DE); DUTZMANN, Stefan, D-40721 Hilden (DE)
(86) Internationale Anmeldenummer: EP9500149
(87) Internationale Veröffentlichungsnummer: WO9520570

(56) Entgegenhaltungen:
- EP-A- 0 463 488
- EP-A- 0 579 124

## Beschreibung

Die Erfindung betrifft neue Hydroxamsäurederivate, ein Verfahren zu ihrer Herstellung und ihre Verwendung als Fungizide.

Bestimmte Hydroxamsäurederivate, wie z.B. die Verbindung N-Hydroxy-α-hydroximino-furazan-ethanimidsäure, sind bereits bekannt (vgl. Liebigs Ann. Chem. 1975, 1029-1050 - zitiert in Chem.Abstracts 88:50732c). Über die biologischen Eigenschaften solcher Verbindungen ist jedoch nichts bekannt geworden.

Es wurden nun die neuen Hydroxamsäurederivate der allgemeinen Formel (I) gefunden, 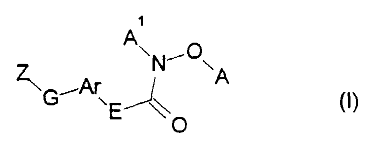 in welcher
- A: für Wasserstoff, für gegebenenfalls durch Halogen, Cyano, Hydroxy. C₁-C₄-Alkoxy, C₁-C₄-Alkoxy-C₁-C₄-alkoxy, N,N-Di-(C₁-C₄-Alkyl)-amino, N-(C₁-C₄-Alkyl-carbonyl)-amino, N-(C₁-C₄-Alkyl)-N-(C₁-C₄-alkyl-carbonyl)-amino; N-(C₁-C₄-Alkoxy-carbonyl)-amino oder N-(C₁-C₄-Alkyl)-N-(C₁-C₄-alkoxy-carbonyl)-amino substituiertes Alkyl mit 1 bis 8 Kohlenstoffatomen, für C₁-C₄-Alkoxy-carbonyl, für C₁-C₄-Alkylamino-carbonyl oder für Di-(C₁-C₄-alkyl)-amino-carbonyl steht,
- A¹: für Wasserstoff oder Alkyl steht,
- Ar: für jeweils gegebenenfalls substituiertes Arylen oder Heteroarylen steht,
- E: für eine 1-Alken-1,1-diyl-Gruppierung steht, die in 2-Position einen Rest R¹ enthält, oder für eine 2-Aza-1-alken-1,1-diyl-Gruppierung steht, die in 2-Position einen Rest R² enthält, oder für eine gegebenenfalls substituierte Imino-Gruppierung ("Azamethylen", N-R³) steht,
wobei
R¹ für Wasserstoff, Halogen, Cyano oder für jeweils gegebenenfalls substituiertes Alkyl, Alkoxy, Alkylthio, Alkylamino oder Dialkylamino steht,
R² für Wasserstoff, Amino, Cyano oder für jeweils gegebenenfalls substituiertes Alkyl, Alkoxy, Alkylamino oder Dialkylamino steht, und
R³ für Wasserstoff Cyano oder für jeweils gegebenenfalls substituiertes Alkyl, Alkenyl, Alkinyl, Cycloalkyl oder Cycloalkylalkyl steht,
- G: für Sauerstoff, für jeweils gegebenenfalls durch Halogen, Hydroxy, Alkyl, Halogenalkyl oder Cycloalkyl substituiertes Alkandiyl, Alkendiyl, Alkindiyl oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O=N-C(R⁴)-, -N(R⁵)-CQ-Q-, -CQ-N(R⁵)-CQ-Q- oder -N(R⁵)-CQ-Q-CH₂- steht,
wobei
n für die Zahlen 0, 1 oder 2 steht,
Q für Sauerstoffoder Schwefel steht,
R⁴ für Wasserstoff, Cyano oder jeweils gegebenenfalls substituiertes Alkyl, Alkoxy, Alkylthio, Alkylamino, Dialkylamino oder Cycloalkyl steht, und
R⁵ für Wasserstoff, Hydroxy, Cyano oder jeweils gegebenenfalls substituiertes Alkyl, Alkoxy oder Cycloalkyl steht, und
- Z: für jeweils gegebenenfalls substituiertes Alkyl, Alkenyl, Alkinyl, Cycloalkyl, Aryl oder Heterocyclyl steht.

Weiter wurde gefunden, daß man die neuen Hydroxamsäurederivate der allgemeinen Formel (I) erhält, wenn man Carbonsäurederivate der allgemeinen Formel (II) 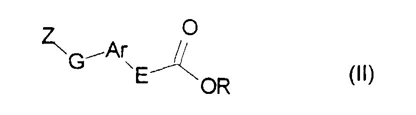 in welcher
- Ar, E, G und Z: die oben angegebene Bedeutung haben und
- R: für Wasserstoff oder Alkyl steht,
mit einem Hydroxylamin der allgemeinen Formel (III)

A¹-NH-O-A (III)

in welcher
- A und A¹: die oben angegebene Bedeutung haben,
- oder mit einem Hydrogenhalogenid hiervon -
gegebenenfalls in Gegenwart eines Säureakzeptors und gegebenenfalls in Gegenwart eines Verdünnungsmittels umsetzt .

Schließlich wurde gefunden, daß die neuen Hydroxamsäurederivate der allgemeinen Formel (I) sehr starke fungizide Wirksamkeit zeigen.

Die erfindungsgemäßen Verbindungen können gegebenenfalls als Mischungen verschiedener möglicher isomerer Formen, insbesondere von E- und Z-Isomeren, gegebenenfalls aber auch von Tautomeren vorliegen. Es werden sowohl die E- als auch die Z-Isomeren wie auch beliebige Mischungen dieser Isomeren sowie die möglichen tautomeren Formen beansprucht.

Gegenstand der Erfindung sind Verbindungen der Formel (I), in welcher
- A: für Wasserstoff, für gegebenenfalls durch Halogen, Cyano, Hydroxy, C₁-C₄-Alkoxy, C₁-C₄-Alkoxy-C₁-C₄-alkoxy, N.N-Di-(C₁-C₄-Alkyl)-amino, N-(C₁-C₄-Alkyl-carbonyl)-amino, N-(C₁-C₄-Alkyl)-N-(C₁-C₄-alkyl-carbonyl)-amino; N-(C₁-C₄-Alkoxy-carbonyl)-amino oder N-(C₁-C₄-Alkyl)-N-(C₁-C₄-alkoxy-carbonyl)-amino substituiertes Alkyl mit 1 bis 8 Kohlenstoffatomen, für C₁-C₄-Alkoxy-carbonyl, für C₁-C₄-Alkylamino-carbonyl oder für Di-(C₁-C₄-alkyl)-amino-carbonyl steht,
- A¹: für Wasserstoff oder Alkyl mit 1 bis 6 Kohlenstoffatomen steht,
- Ar: für jeweils gegebenenfalls substituiertes Phenylen oder Naphthylen oder für Heteroarylen mit 5 oder 6 Ringgliedern, von denen mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
- E: für eine der nachstehenden Gruppierungen steht 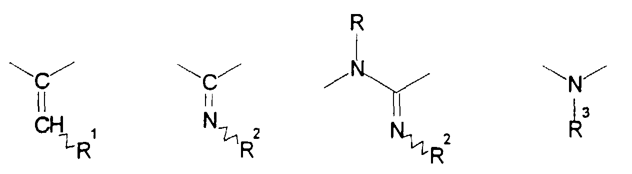 worin
R für Wasserstoff oder Alkyl mit 1 bis 6 Kohlenstoffatomen steht,
R¹ für Wasserstoff, Halogen, Cyano oder für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylresten steht,
R² für Wasserstoff, Amino, Cyano oder für jeweils gegebenenfalls durch Halogen, Cyano oder C₁-C₄-Alkoxy substituiertes Alkyl, Alkoxy, Alkylamino oder Dialkylamino mit jeweils 1 bis 6 Kohlenstoffatomen in den Alkylresten steht, und
R³ für Wasserstoff, Cyano oder für jeweils gegebenenfalls durch Halogen, Cyano oder C₁-C₄-Alkoxy substituiertes Alkyl, Alkenyl oder Alkinyl mit jeweils bis zu 6 Kohlenstoffatomen oder für jeweils gegebenenfalls durch Halogen, Cyano, Carboxy, C₁-C₄-Alkyl oder C₁-C₄-Alkoxy-carbonyl substituiertes Cycloalkyl oder Cycloalkylalkyl mit 3 bis 6 Kohlenstoffatomen in den Cycloalkylteilen und gegebenenfalls 1 bis 4 Kohlenstoffatomen im Alkylteil steht,
- G: für Sauerstoff, für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- oder -N(R⁵)-CQ-Q-CH₂- steht,
wobei
n für die Zahlen 0, 1 oder 2 steht,
Q für Sauerstoff oder Schwefel steht,
R⁴ für Wasserstoff, Cyano, für gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
R⁵ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
- Z: für gegebenenfalls durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) substituiertes Alkyl mit 1 bis 8 Kohlenstoffatomen, für jeweils gegebenenfalls durch Halogen substituiertes Alkenyl oder Alkinyl mit jeweils bis zu 8 Kohlenstoffatomen, für jeweils gegebenenfalls durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen, für jeweils gegebenenfalls substituiertes Phenyl, Naphthyl oder für Heterocyclyl mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, Cycloalkyl mit 3 bis 6 Kohlenstoffatomen, Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -, sowie jeweils gegebenenfalls im Phenylteil einfach oder mehrfach, gleich oder verschieden durch Halogen, Cyano und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen substituiertes Phenyl, Phenoxy, Benzyl, Benzyloxy, Phenylethyl oder Phenylethyloxy.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- A: für Wasserstoff, für gegebenenfalls durch Fluor, Chlor, Methoxy, Ethoxy, n-oder i-Propoxy, Methoxy-ethoxy, Ethoxy-ethoxy, Dimethylamino, Diethylamino, Acetylamino, Propionylamino, N-Methyl-acetylamino, N-Ethyl-acetylamino, N-Methyl-propionylamino, N-Ethyl-propionylamino, Methoxycarbonylamino, Ethoxycarbonylamino, N-Methyl-N-methoxycarbonylamino, N-Ethyl-N-methoxycarbonylamino, N-Methyl-N-ethoxycarbonylamino oder N-Ethyl-N-ethoxycarbonylamino substituiertes Methyl, Ethyl, n- oder i-Propyl, n-, i-, s- oder t-Butyl, für Methoxycarbonyl, Ethoxycarbonyl, Methylaminocarbonyl, Ethylaminocarbonyl, Dimethylaminocarbonyl oder Diethylaminocarbonyl steht
- A¹: für Wasserstoff, Methyl, Ethyl, n- oder i-Propyl, n-, i- oder s-Butyl steht,
- Ar: für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Trifluormethyl, Methoxy, Ethoxy, Methylthio. Methylsulfinyl oder Methylsulfonyl,
- E: für eine der nachstehenden Gruppierungen steht 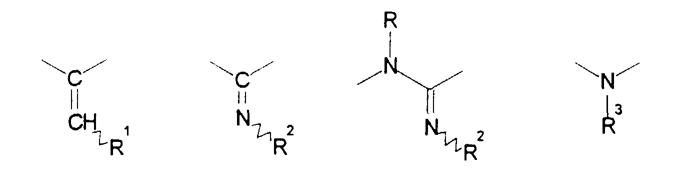 worin
R für Methyl, Ethyl, n- oder i-Propyl, n-, i- oder s-Butyl steht,
R¹ für Wasserstoff, Fluor, Chlor, Brom, Cyano oder für jeweils gegebenenfalls durch Fluor, Chlor, Cyano, Methoxy oder Ethoxy substituiertes Methyl, Ethyl, Propyl, Methoxy, Ethoxy, Methylthio, Ethylthio, Methylamino, Ethylamino oder Dimethylamino steht,
R² für Wasserstoff Amino, Cyano oder für jeweils gegebenenfalls durch Fluor, Chlor, Cyano, Methoxy oder Ethoxy substituiertes Methyl, Ethyl, Methoxy, Ethoxy, Methylamino, Ethylamino oder Dimethylamino steht, und
R³ für Wasserstoff, Cyano oder für jeweils gegebenenfalls durch Fluor, Cyano, Methoxy oder Ethoxy substituiertes Methyl, Ethyl n- oder i-Propyl, n-, i- oder s-Butyl, für Allyl oder Propargyl oder für jeweils gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
- G: für Sauerstoff, für jeweils gegebenenfalls durch Fluor, Chlor, Hydroxy, Methyl, Ethyl, n- oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- oder -N(R⁵)-CQ-Q-CH₂- steht,
wobei
n für die Zahlen 0, 1 oder 2 steht,
Q für Sauerstoff oder Schwefel steht,
R⁴ für Wasserstoff, Cyano, für gegebenenfalls durch Fluor, Chlor, Cyano, Methoxy oder Ethoxy substituiertes Methyl, Ethyl, n- oder i-Propyl, n-, i- oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und
R⁵ für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht, und
- Z: für gegebenenfalls durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl, für jeweils gegebenenfalls durch Fluor, Chlor oder Brom substituiertes Allyl, Crotonyl, 1-Methyl-allyl, Propargyl oder 1-Methyl-propargyl , für jeweils gegebenenfalls durch Fluor, Chlor, Brom, Cyano, Carboxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substiuiertes Cyclopropyl, Cyclobutyl, Cyclopentyl oder Cyclohexyl, für jeweils gegebenenfalls substituiertes Phenyl, Naphthyl, Furyl, Thienyl, Oxazolyl, Isoxazolyl, Thiazolyl, Isothiazolyl, Oxadiazolyl, Thiadiazolyl, Pyridinyl, Pyrimidinyl, Pyridazinyl, Pyrazinyl, 1,2,3-Triazinyl, 1,2,4-Triazinyl, 1,3,5-Triazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Difluormethoxy, Trifluormethoxy, Difluormethylthio, Trifluormethylthio, Trifluormethylsulfinyl oder Trifluormethylsulfonyl , Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Ethyl, n- oder i-Propyl substituiertes Trimethylen (Propan-1,3-diyl), Methylendioxy oder Ethylendioxy, Cyclopropyl, Cyclobutyl, Cyclopentyl oder Cyclohexyl, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy.

Eine besonders bevorzugte Gruppe erfindungsgemäßer Verbindungen sind diejenigen Verbindungen der Formel (I), in welcher
- A: für Wasserstoff steht,
- A¹: für Wasserstoff oder Methyl steht,
- Ar: für ortho-Phenylen, Pyridin-2,3-diyl oder Thiophen-2,3-diyl steht,
- E: für eine der nachstehenden Gruppierungen steht 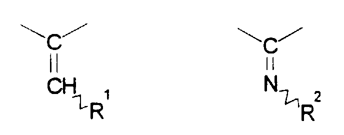 worin
R¹ und R² jeweils für Methoxy stehen,
- G: für Sauerstoff, Methylen oder eine der nachstehenden Gruppierungen -CH₂-O-, -O-CH₂-, -S(O)ₙ-, -CH₂-S(O)ₙ-, -S(O)ₙ-CH₂-, -C(R⁴)=N-O-, -O-N=C(R⁴)-, -C(R⁴)=N-O-CH₂-, -N(R⁵)- oder -CH₂-O-N=C(R⁴)- steht,
wobei
n für die Zahlen 0, 1 oder 2 steht,
R⁴ für Wasserstoff, Methyl oder Ethyl steht und
R⁵ für Wasserstoff, Methyl oder Ethyl steht, und
- Z: für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i- Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise α-Methoximino-α-(2-phenoxymethyl-phenyl)-essigsäure-methylester und Hydroxylamin-Hydrochlorid als Ausgangsstoffe, so kann der Reaktionsablauf beim erfindungsgemäßen Herstellungsverfahren durch das folgende Formelschema skizziert werden:

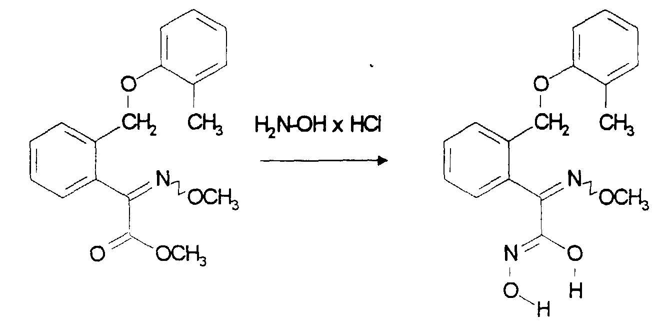

Die zur Durchführung des erfindungsgemäßen Verfahrens als Ausgangsstoffe benötigten Carbonsäurederivate sind durch die Formel (II) allgemein definiert. In dieser Formel (II) haben Ar, E, G und Z vorzugsweise bzw. insbesondere diejenige Bedeutungen, die bereits im Zusammenhang mit der Beschreibung der erfindungsgemäßen Verbindungen der Formel (I) als bevorzugt bzw. als insbesondere bevorzugt für Ar, E, G und Z angegeben wurden; R steht vorzugsweise für Alkyl mit 1 bis 4 Kohlenstoffatomen, insbesondere für Methyl oder Ethyl.

Die Ausgangsstoffe der Formel (II) sind bekannt und/oder können nach an sich bekannten Verfahren hergestellt werden (vgl. EP-A 178826, EP-A 242081, EP-A 382375, EP-A 493711).

Die weiter als Ausgangsstoffe zu verwendenden Hydroxylamine sind durch die Formel (III) allgemein definiert. In der Formel (III) hat A vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für A angegeben wurde.

Die Ausgangsstoffe der Formel (III) sind bekannt organische Synthesechemikalien.

Das erfindungsgemäße Verfahren wird vorzugsweise in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens kommen Wasser und organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol. Petrolether. Hexan. Cyclohexan. Dichlormethan, Chloroform, Tetrachlorkohlenstoff, Ether, wie Diethylether, Diisopropylether, Dioxan, Tetrahydrofuran oder Ethylenglykol-dimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen -20°C und +100°C, vorzugsweise bei Temperaturen zwischen 0°C und 50°C.

Zur Durchführung des erfindungsgemäßen Verfahrens setzt man je Mol an Carbonsäurederivat der Formel (II) im allgemeinen 1 bis 5 Mol, vorzugsweise 1,0 bis 2,5 Mol an Hydroxylamin oder Hydroxylamin-Hydrogenhalogenid und gegebenenfalls 1 bis 10 Mol, vorzugsweise 1 bis 5 Mol an Säureakzeptor ein.

Die Reaktionsdurchführung, Aufarbeitung und Isolierung der Reaktionsprodukte erfolgt nach bekannten Verfahren (vgl. die Herstellungsbeispiele).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen Phytophthora- und Venturia-Arten, oder zur Bekämpfung von Getreidekrankheiten, wie beispielsweise gegen Erysiphe-Arten sowie zur Bekämpfung von Reiskrankheiten, wie beispielsweise gegen Pyricularia oryzae, eingesetzt werden.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen treten dabei synergistische Effekte auf.

Für die Mischungen kommen beispielsweise infrage:

**Fungizide:**
2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyano-phenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol, Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung, Mancopper, Mancozeb, Maneb, Mepanipyrim, Mepronil, Metalaxyl, Metconazol. Methasulfocarb, Methfuroxam, Metiram, Metsulfovax, Myclobutanil, Nickel dimethyldithiocarbamat. Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

**Bakterizide:**
Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**
Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon. Dichlofenthion. Dichlorvos, Didiphos, Dicrotophos, Diethion, Diflubenzuron. Dimethoat, Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb, HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, ,Primiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen. Spritzpulver, Pasten, losliche Pulver, Stäubemittel und Granulate angewendet werden.

Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele:

### Beispiel 1

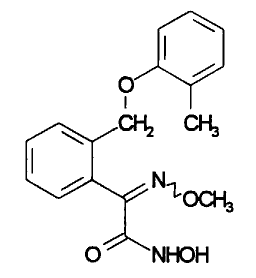

2,67 g (38,4 mMol) Hydroxylamin-Hydrochlorid werden in 40 ml Methanol vorgelegt und bei 10°C werden 6,0 g (19,2 mMol) α-Methoximino-α-[2-(2-methyl-phenoxy-methyl)-phenyl]-essigsäure-methylester portionsweise dazu gegeben. Dann werden unter Eiskühlung 13,8 g einer 30%igen Natriummethylatlösung (76,8 mMol NaOCH₃) tropfenweise innerhalb von 15 Minuten zur Mischung gegeben und das Reaktionsgemisch wird anschließend nach Entfernen des Eisbades 3 Stunden bei 0°C bis 20°C gerührt.

Zur Aufarbeitung wird der Ansatz in verdünnte Salzsäure (aus 6 ml konz. Salzsäure und 100 g Eiswasser hergestellt) eingerührt, dann wird mit Essigsäureethylester geschüttelt, die organische Phase über Natriumsulfat getrocknet und filtriert. Vom Filtrat wird das Lösungsmittel im Wasserstrahlvakuum sorgfältig abdestilliert.

Man erhält 5,3 g (81% der Theorie) α-Methoximino-α-[2-(2-methyl-phenoxymethyl)-phenyl]-acethydroxamsäure (92%ig nach "Liquid Chromatography") vom Schmelzpunkt 65°C.

¹H-NMR(D₆-Dimethylsulfoxid,δ): 2,20, 3,88, 3,95, 6,8-7,55, 9,2 ppm.

Analog Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

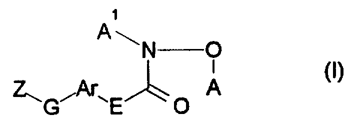

Die ¹H-NMR-Spektren wurden aufgenommen in CDCl₃ oder in D₆-Dimethylsulfoxid mit Tetramethylsilan als innerem Standard. Angegeben sind in der Regel die chemischen Verschiebungen als δ-Werte.

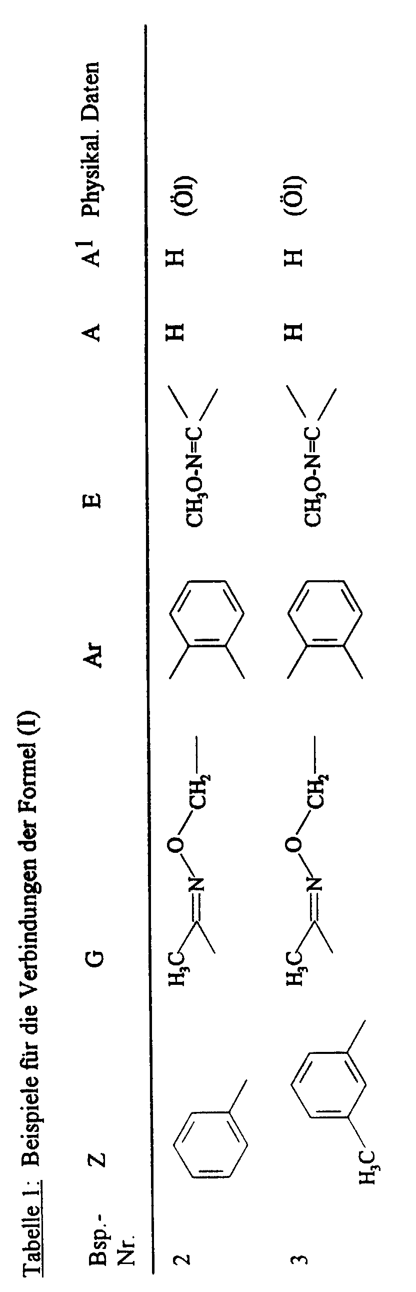

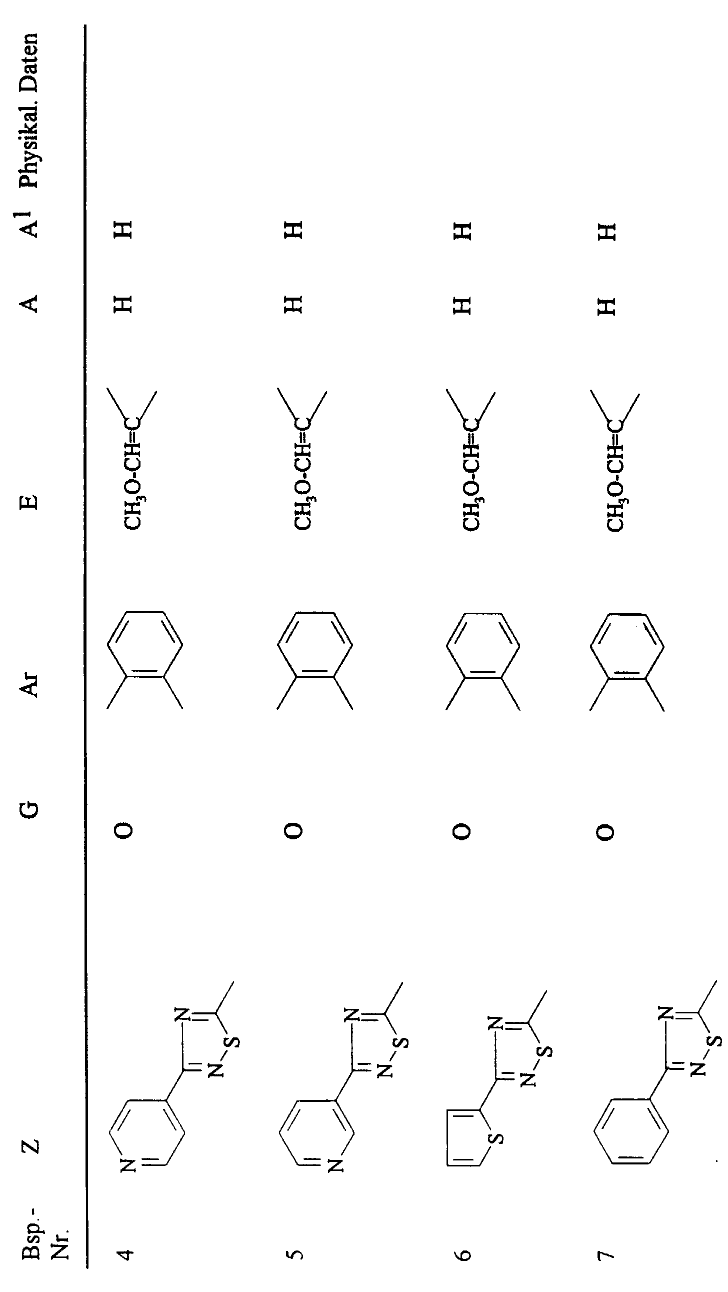

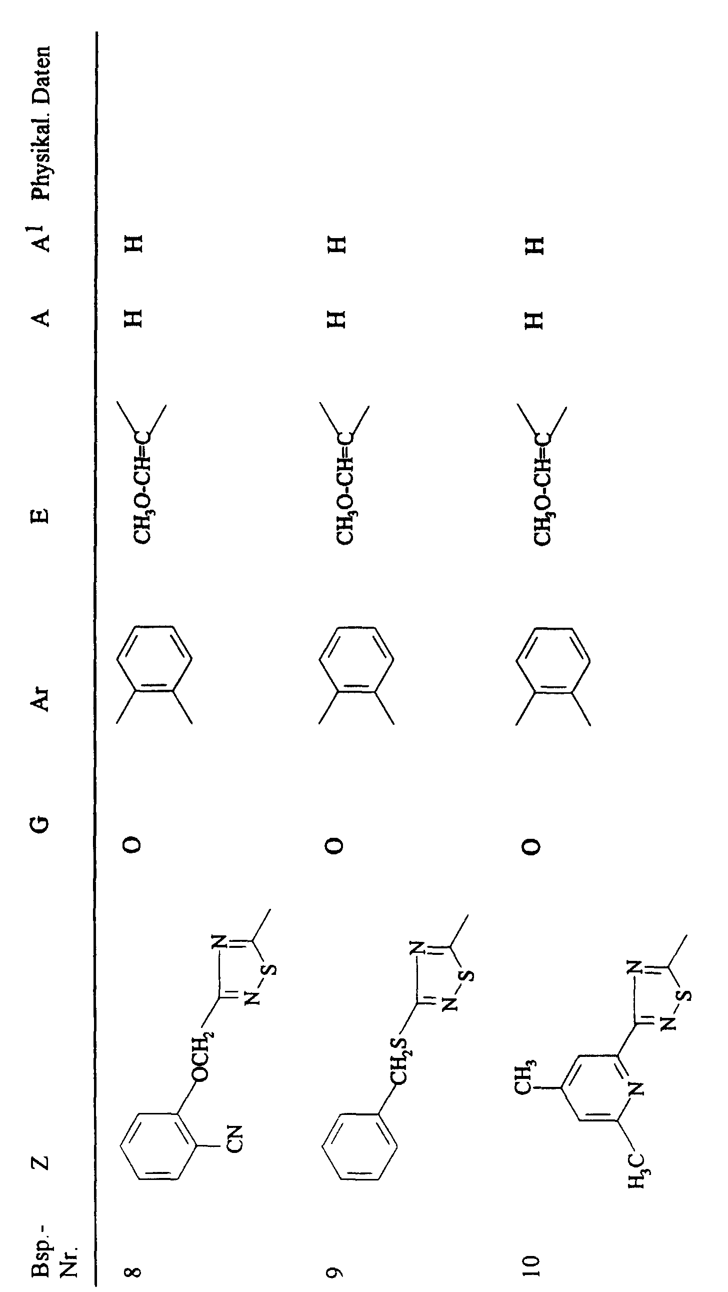

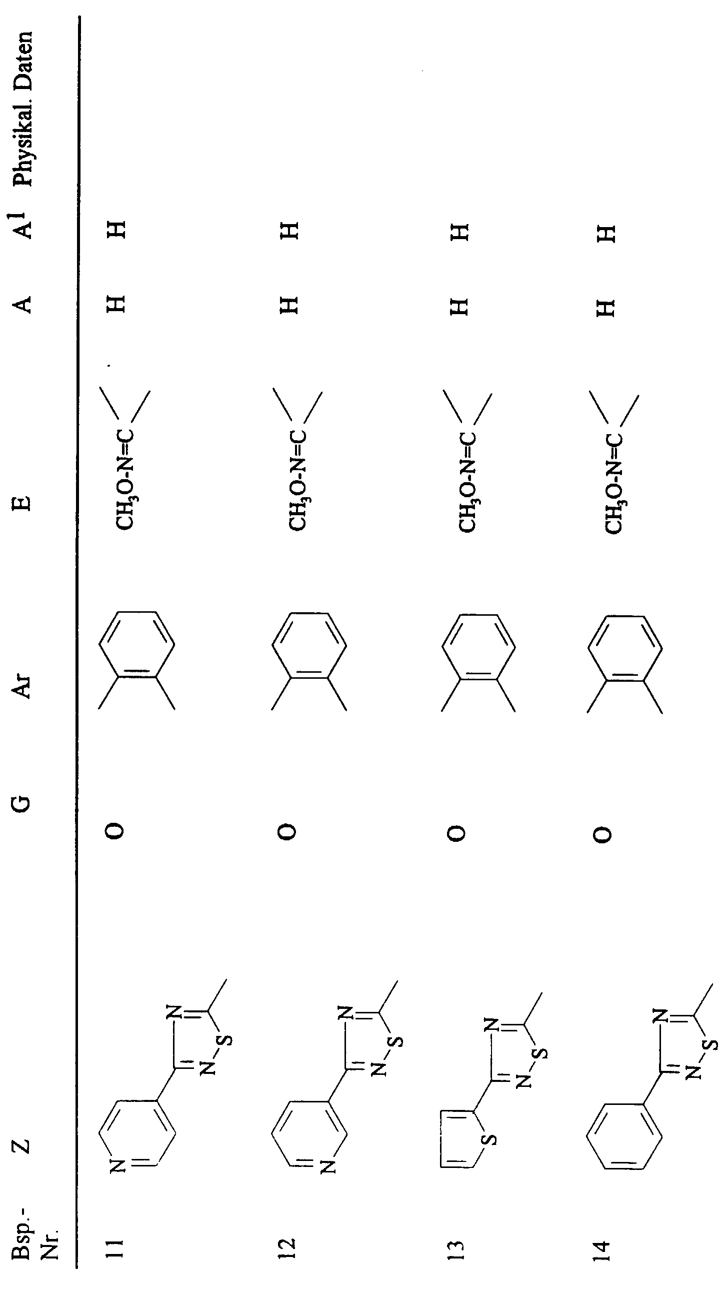

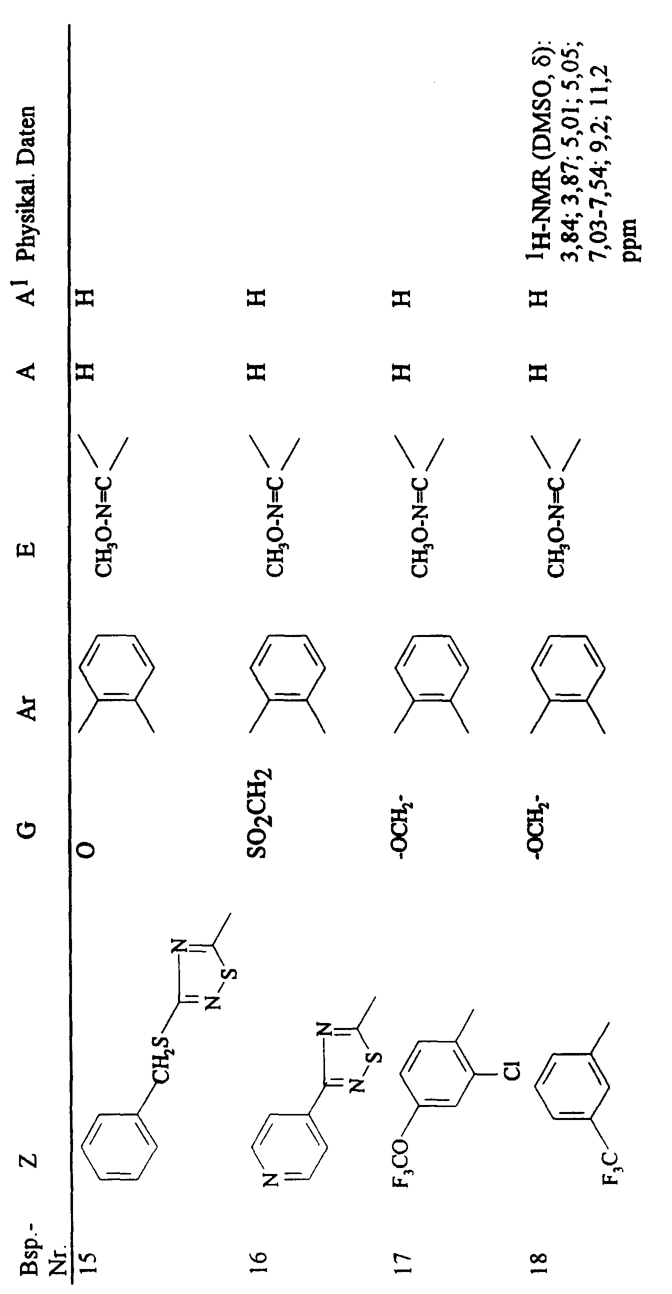

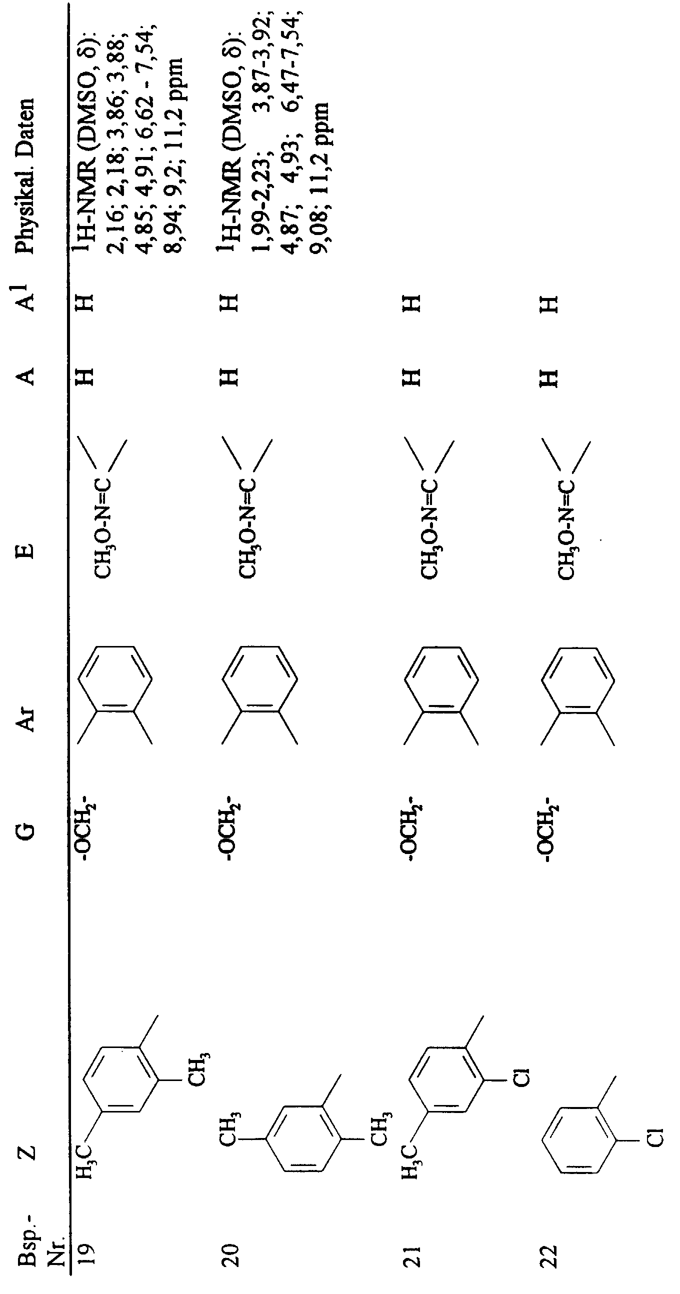

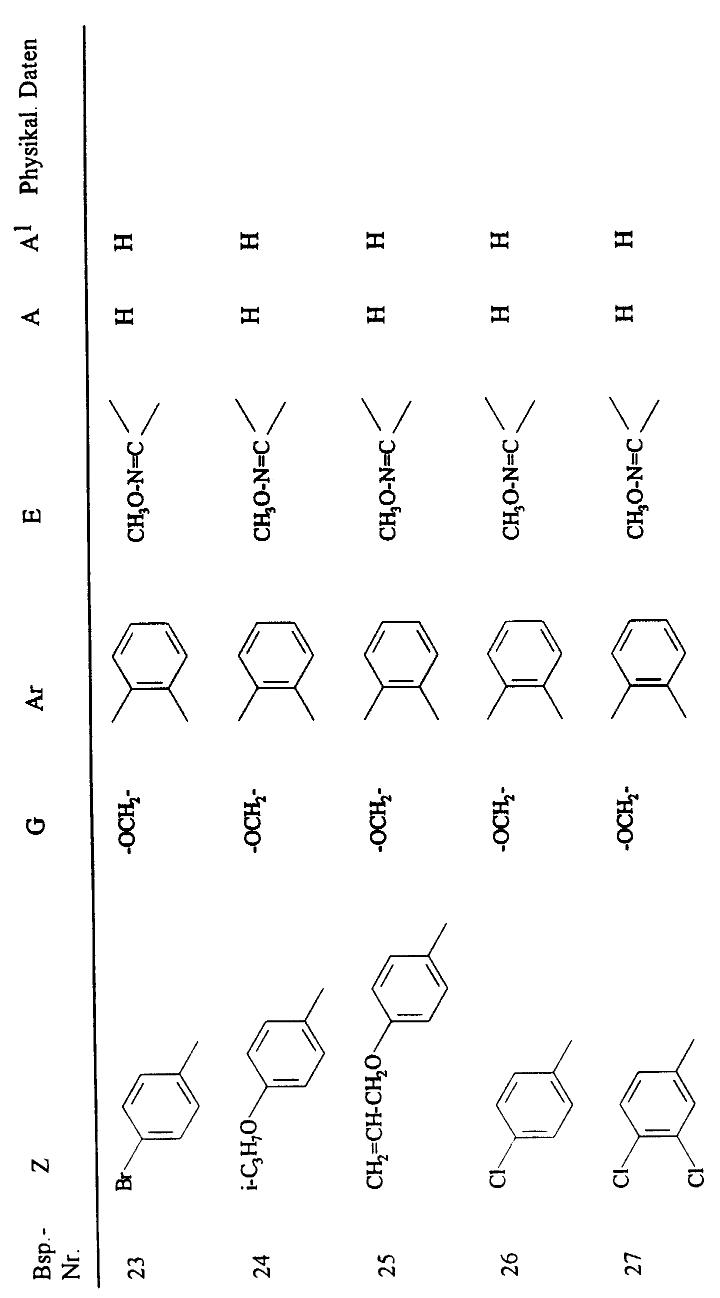

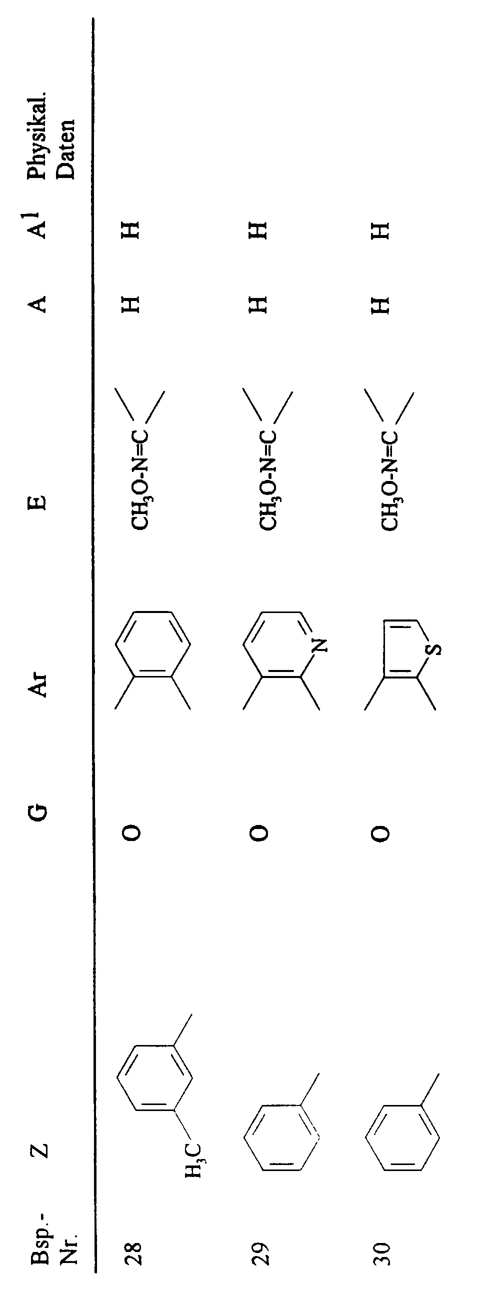

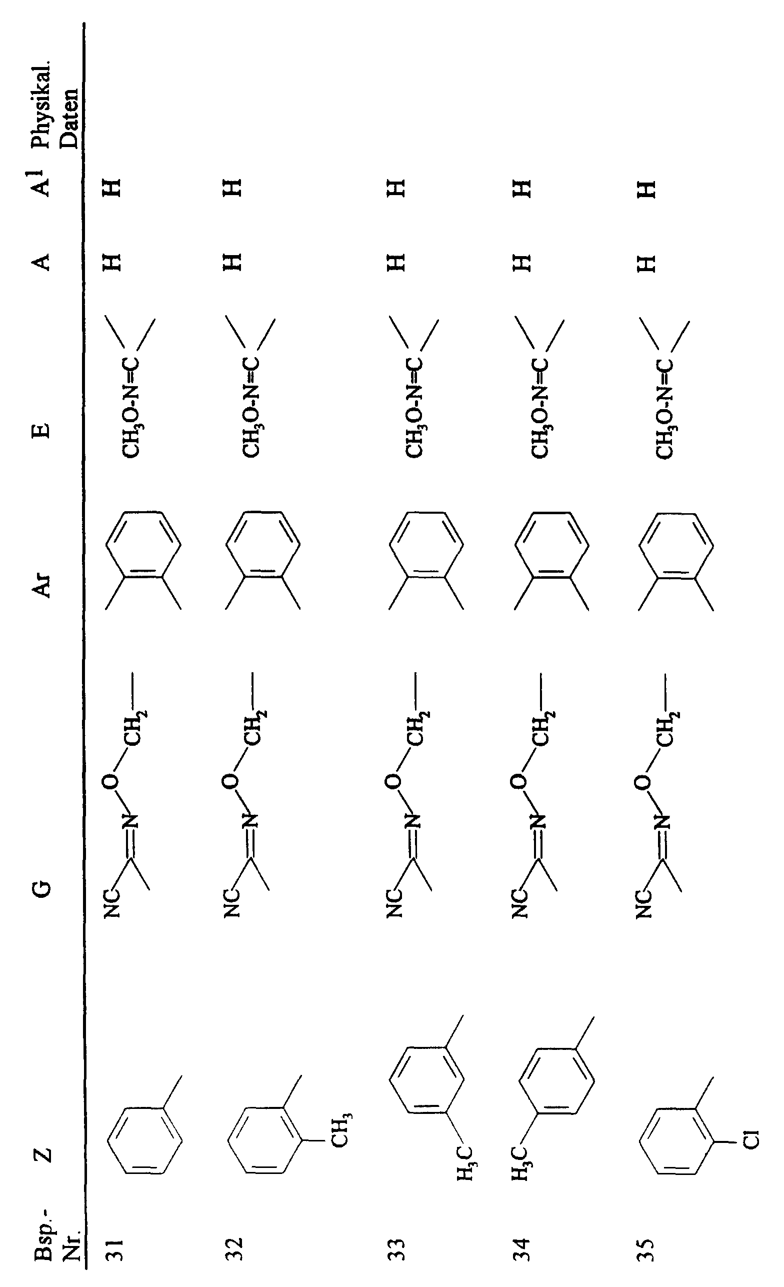

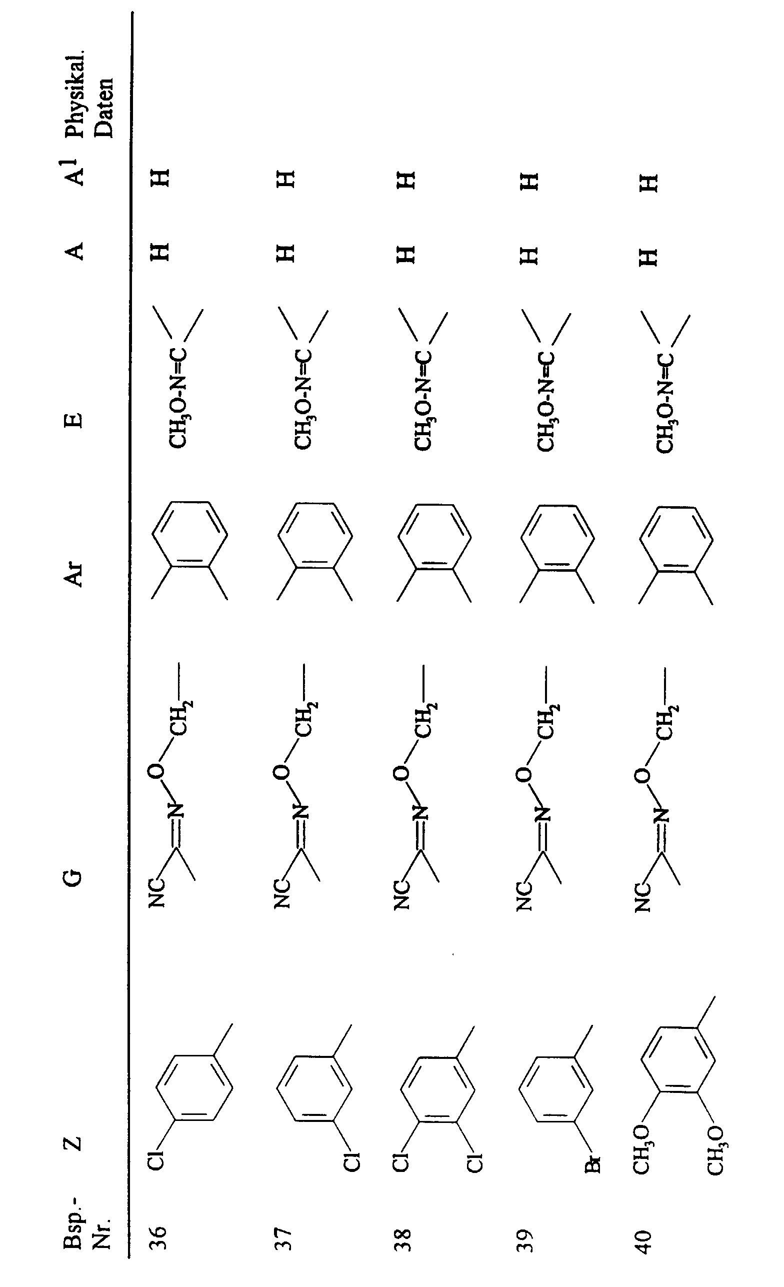

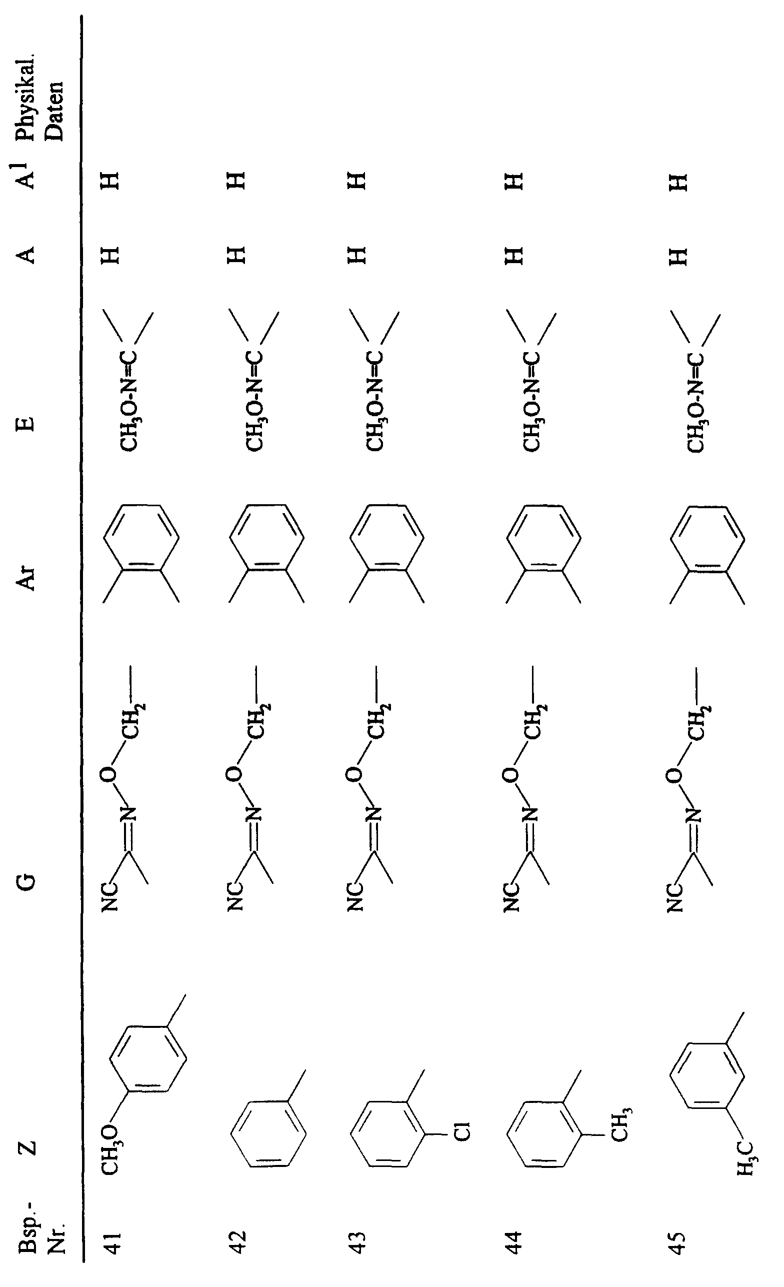

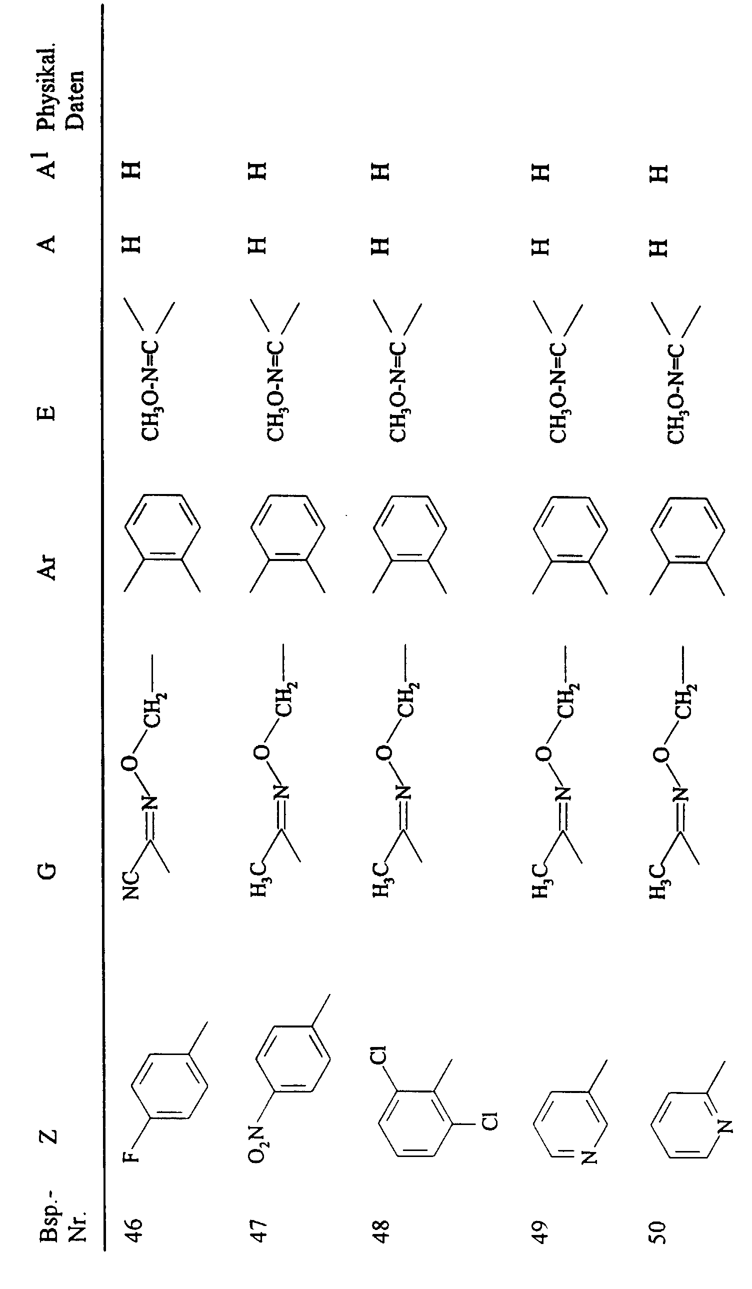

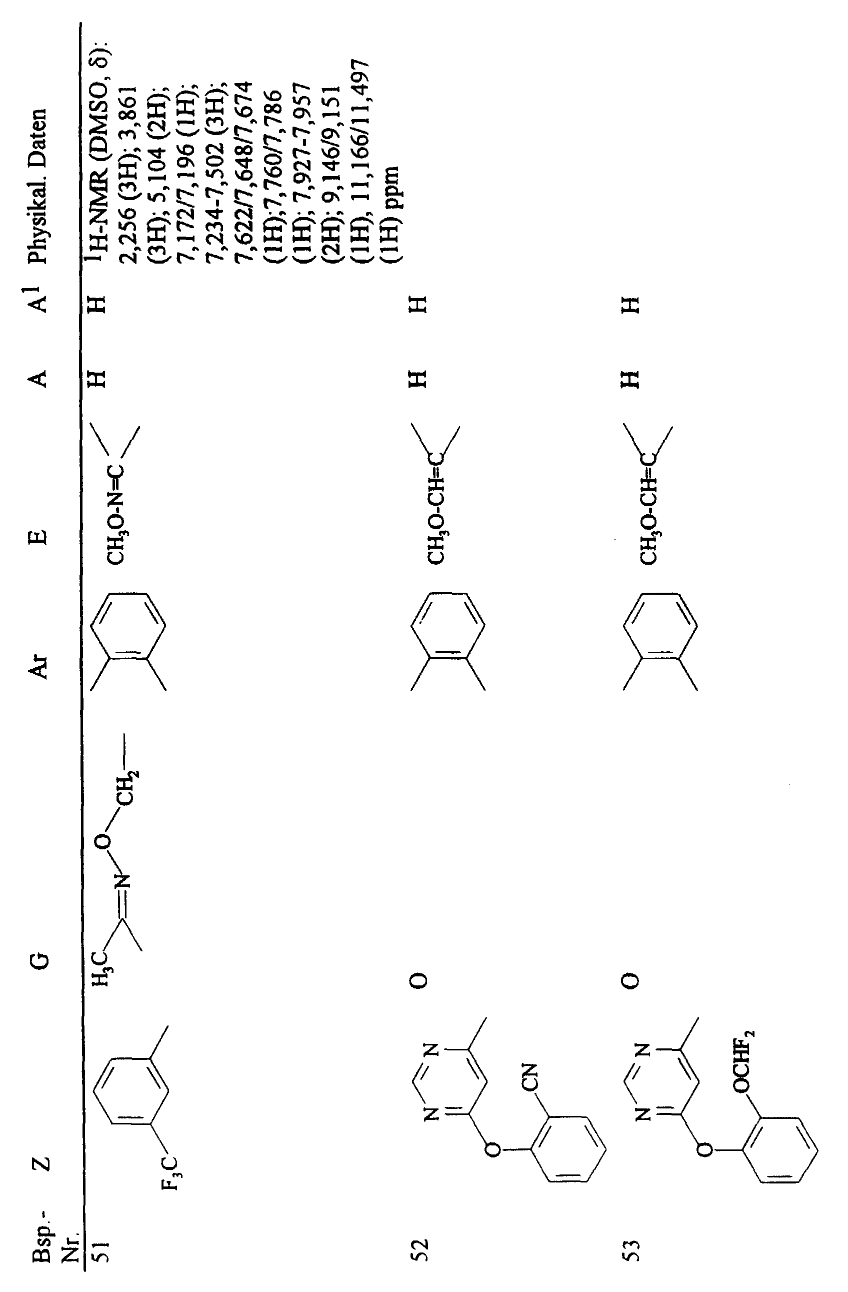

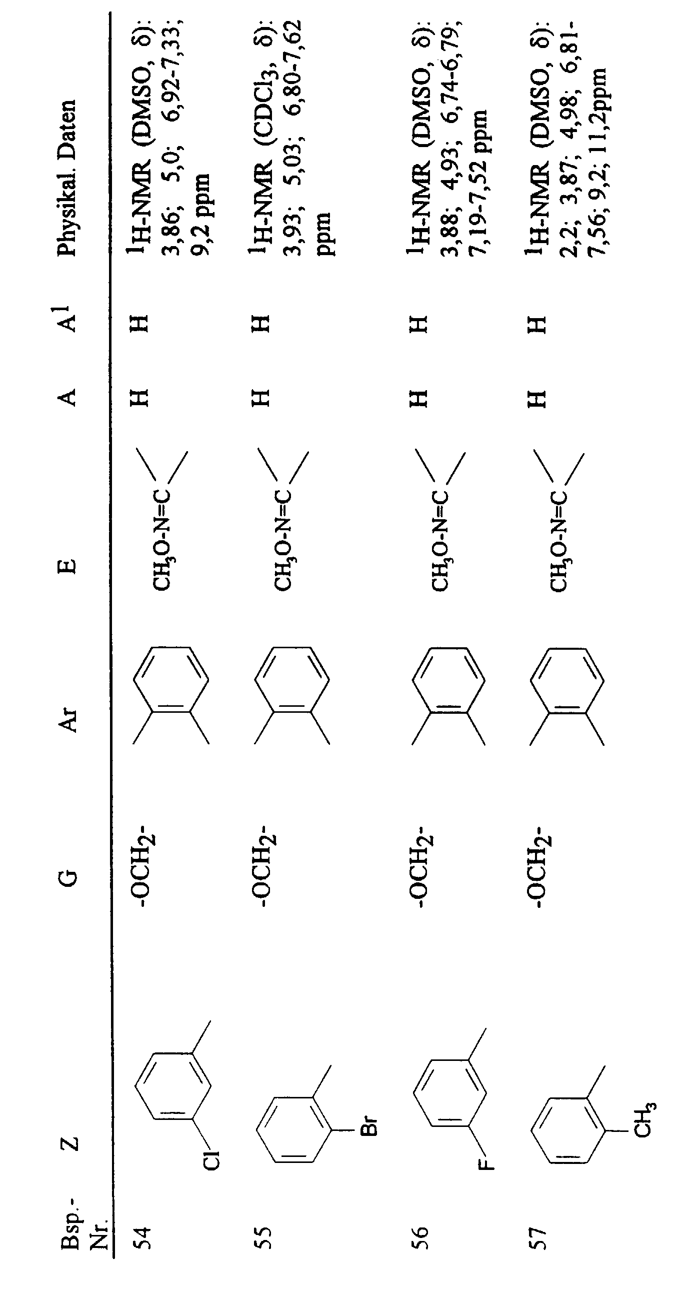

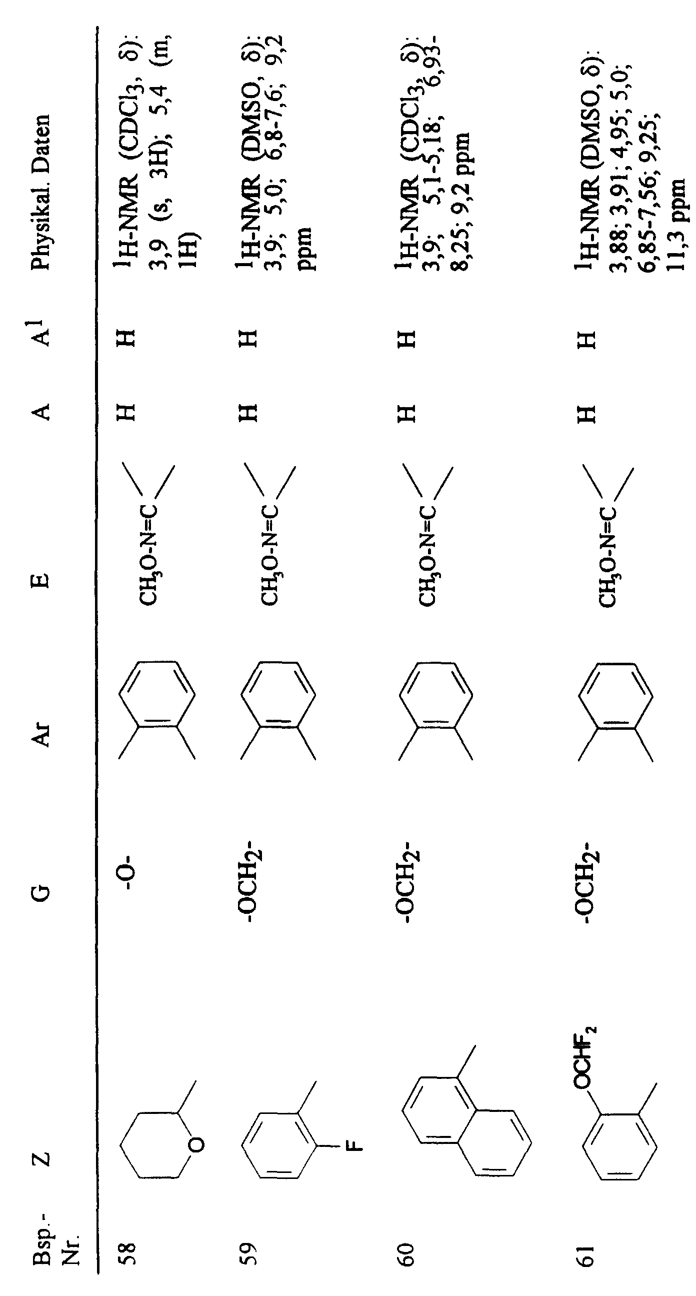

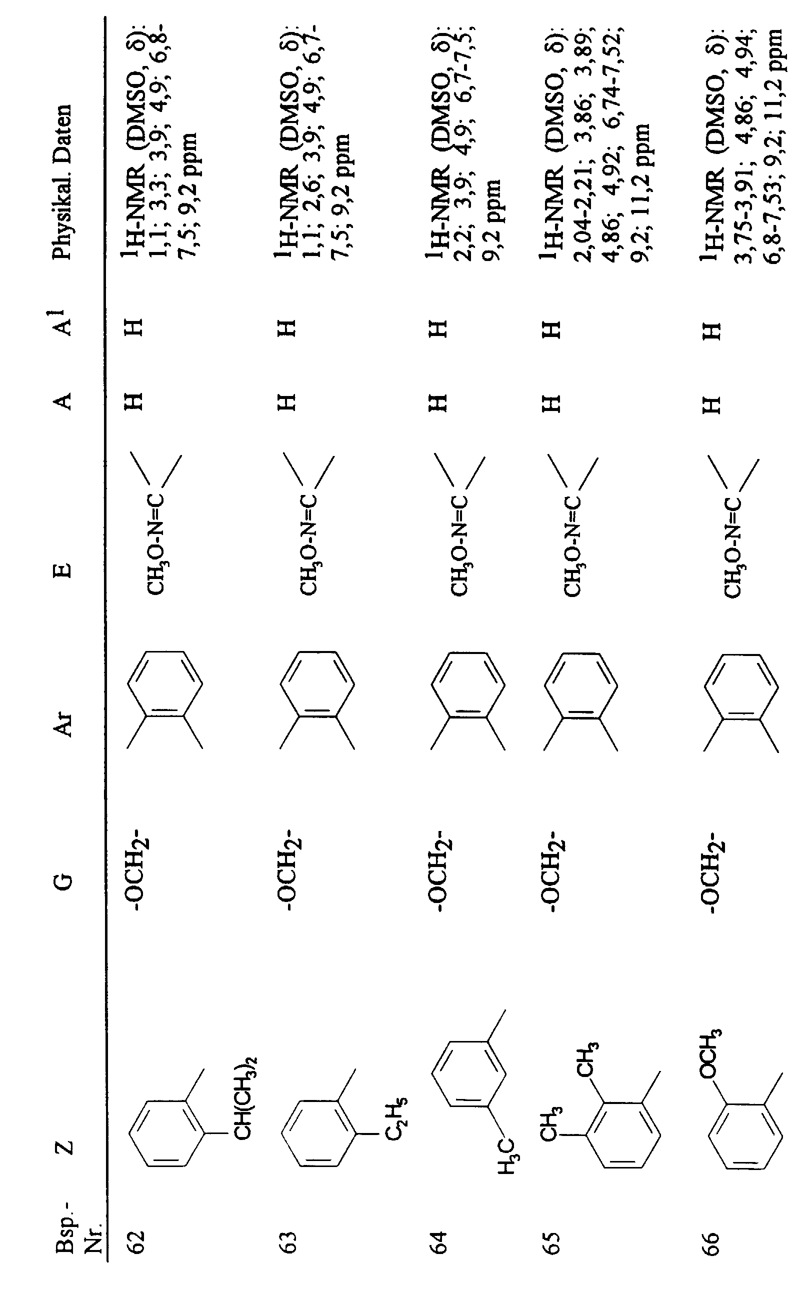

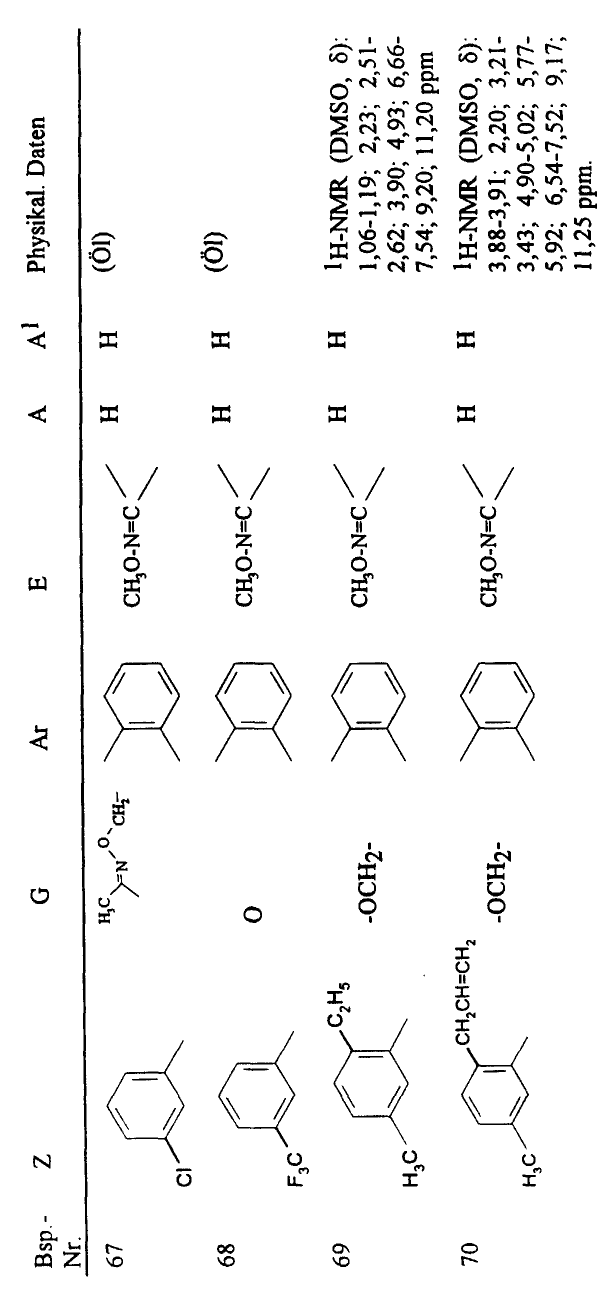

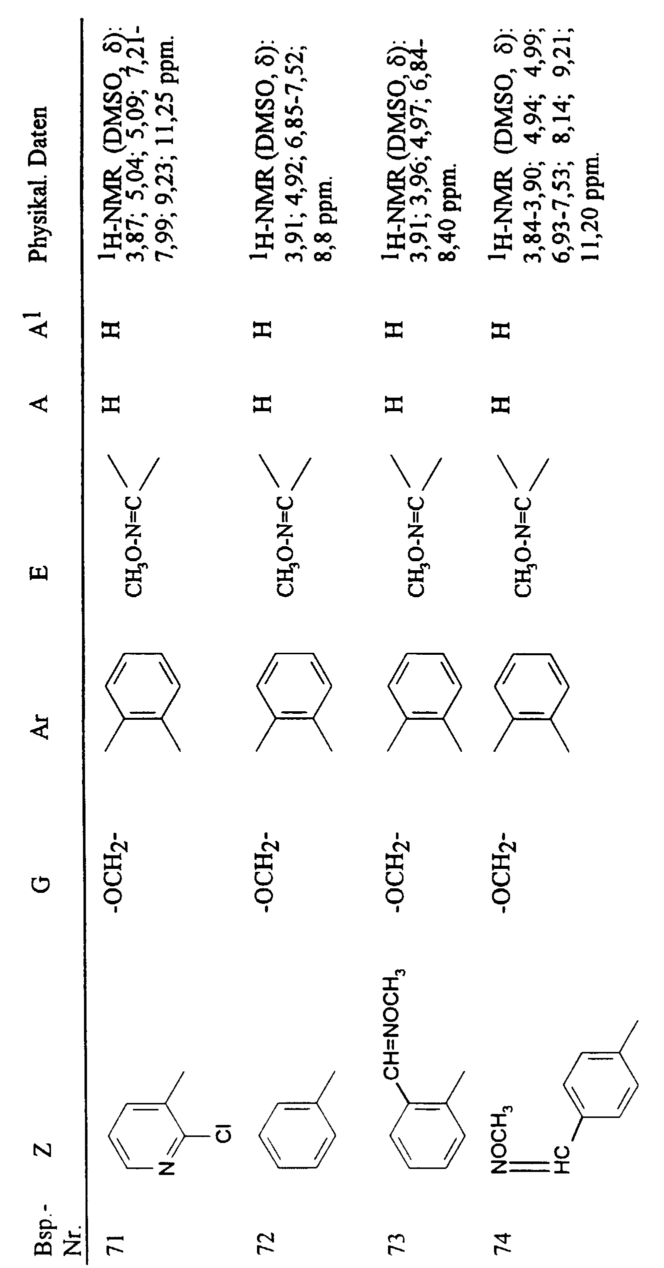

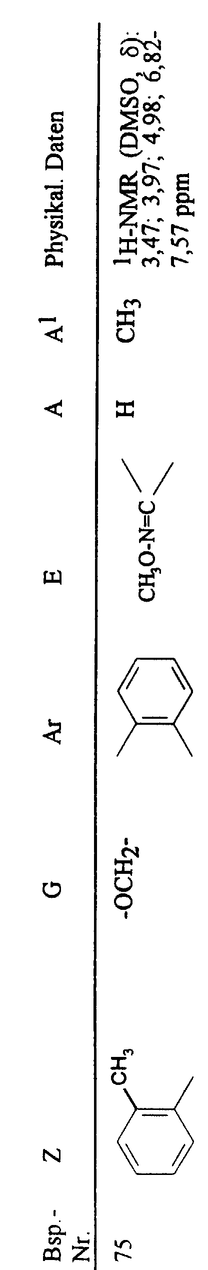

### Anwendungsbeispiele:

### Beispiel A

| | | |
|---|---|---|
| **Pyricularia-Test (Reis)** / protektiv | | |
| Lösungsmittel | 12,5 | Gewichtsteile Aceton |
| Emulgator | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von *Pyricularia oryzae* inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

In diesem Test zeigte beispielsweise die Verbindung gemäß Herstellungsbeispiel 1 bei einer Wirkstoffkonzentration von 0,025% einen Wirkungsgrad von 100%.

### Beispiel B

| | | |
|---|---|---|
| **Pyricularia-Test (Reis)** / systemisch | | |
| Lösungsmittel | 12,5 | Gewichtsteile Aceton |
| Emulgator | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von *Pyricularia oryzae* inokuliert. Danach verbleiben die Pflanzen in einem Gewächshaus bei einer Temperatur von 25°C und einer rel. Luftfeuchtigkeit von 100% bis zur Auswertung.

4 Tage nach der Inokulation erfolgt die Auswertung des Krankheitsbefalls.

In diesem Test zeigte beispielsweise die Verbindung gemäß Herstellungsbeispiel 1 bei einer Aufwandmenge von 100 mg pro 100 cm² einen Wirkungsgrad von 67%.

### Beispiel C

| | |
|---|---|
| **Phytophthora-Test** (Tomate) / protektiv | |
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von *Phytophthora infestans* inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

In diesem Test zeigte beispielsweise die Verbindung gemäß Herstellungsbeispiel 1 bei einer Wirkstoffkonzentration von 0,025% einen Wirkungsgrad von 67%.

### Beispiel D

| | | |
|---|---|---|
| **Erysiphe-Test (Gerste)** / protektiv | | |
| Lösungsmittel | 12,5 | Gewichtsteile N-Methyl-pyrrolidon |
| Emulgator | 0,3 | Gewichtsteile Alkylarylpolyglykolether |

Zur Herstellung einer zweckwäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von *Erisiphe graminis f.sp.hordei* bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigte beispielsweise die Verbindung gemäß Herstellungsbeispiel 1 bei einer Wirkstoffkonzentration von 0,025% einen Wirkungsgrad von 89%.

### Beispiel E

| | |
|---|---|
| **Venturia-Test (Apfel)** / protektiv | |
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers *Venturia inaequalis* inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

In diesem Test zeigte beispielsweise die Verbindung gemäß Herstellungsbeispiel 1 bei einer Wirkstoffkonzentration von 0,025% einen Wirkungsgrad von 100%.

## Patentansprüche

1. Hydroxamsäurederivate der allgemeinen Formel (I), 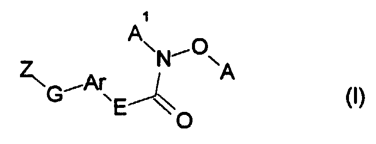 in welcher
A für Wasserstoff, für gegebenenfalls durch Halogen, Cyano, Hydroxy, C₁-C₄-Alkoxy, C₁-C₄-Alkoxy-C₁-C₄-alkoxy, N,N-Di-(C₁-C₄-Alkyl)-amino, N-(C₁-C₄-Alkyl-carbonyl)-amino, N-(C₁-C₄-Alkyl)-N-(C₁-C₄-alkyl-carbonyl)-amino; N-(C₁-C₄-Alkoxy-carbonyl)-amino oder N-(C₁-C₄-Alkyl)-N-(C₁-C₄-alkoxy-carbonyl)-amino substituiertes Alkyl mit 1 bis 8 Kohlenstoffatomen, für C₁-C₄-Alkoxy-carbonyl, für C₁-C₄-Alkylamino-carbonyl oder für Di-(C₁-C₄-alkyl)-amino-carbonyl steht,
A¹ für Wasserstoff oder Akyl steht,
Ar für jeweils gegebenenfalls substituiertes Arylen oder Heteroarylen steht,
E für eine 1-Alken-1,1-diyl-Gruppierung steht, die in 2-Position einen Rest R¹ enthält, oder für eine 2-Aza-1-alken-1,1-diyl-Gruppierung steht, die in 2-Position einen Rest R² enthält, oder für eine gegebenenfalls substituierte Imino-Gruppierung ("Azamethylen", N-R³) steht,
wobei
R¹ für Wasserstoff, Halogen, Cyano oder für jeweils gegebenenfalls substituiertes Alkyl, Alkoxy, Alkylthio, Alkylamino oder Dialkylamino steht,
R² für Wasserstoff, Amino, Cyano oder für jeweils gegebenenfalls substituiertes Alkyl, Alkoxy, Alkylamino oder Dialkylamino steht, und
R³ für Wasserstoff, Cyano oder für jeweils gegebenenfalls substituiertes Alkyl, Alkenyl, Alkinyl, Cycloalkyl oder Cycloalkylalkyl steht,
G für Sauerstoff, für jeweils gegebenenfalls durch Halogen, Hydroxy, Alkyl, Halogenalkyl oder Cycloalkyl substituiertes Alkandiyl, Alkendiyl, Alkindiyl oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O=N-C(R⁴)-, -N(R⁵)-CQ-Q-, -CQ-N(R⁵)-CQ-Q- oder -N(R⁵)-CQ-Q-CH₂- steht,
wobei
n für die Zahlen 0, 1 oder 2 steht,
Q für Sauerstoffoder Schwefel steht,
R⁴ für Wasserstoff, Cyano oder jeweils gegebenenfalls substituiertes Alkyl, Alkoxy, Alkylthio, Alkylamino, Dialkylamino oder Cycloalkyl steht, und
R⁵ für Wasserstoff, Hydroxy, Cyano oder jeweils gegebenenfalls substituiertes Alkyl, Alkoxy oder Cycloalkyl steht, und
Z für jeweils gegebenenfalls substituiertes Alkyl, Alkenyl, Alkinyl, Cycloalkyl, Aryl oder Heterocyclyl steht.

2. Verbindungen der Formel (I) nach Anspruch 1, in welcher
A für Wasserstoff, für gegebenenfalls durch Halogen, Cyano, Hydroxy, C₁-C₄-Alkoxy, C₁-C₄-Alkoxy-C₁-C₄-alkoxy, N,N-Di-(C₁-C₄-Alkyl)-amino, N-(C₁-C₄-Alkyl-carbonyl)-amino, N-(C₁-C₄-Alkyl)-N-(C₁-C₄-alkyl-carbonyl)-amino; N-(C₁-C₄-Alkoxy-carbonyl)-amino oder N-(C₁-C₄-Alkyl)-N-(C₁-C₄-alkoxy-carbonyl)-amino substituiertes Alkyl mit 1 bis 8 Kohlenstoffatomen, für C₁-C₄-Alkoxy-carbonyl, für C₁-C₄-Alkylamino-carbonyl oder für Di-(C₁-C₄-alkyl)-amino-carbonyl steht,
A¹ für Wasserstoff oder Alkyl mit 1 bis 6 Kohlenstoffatomen steht,
Ar für jeweils gegebenenfalls substituiertes Phenylen oder Naphthylen oder für Heteroarylen mit 5 oder 6 Ringgliedern, von denen mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
E für eine der nachstehenden Gruppierungen steht 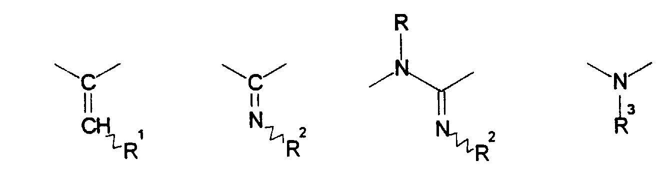 worin
R für Wasserstoff oder Alkyl mit 1 bis 6 Kohlenstoffatomen steht,
R¹ für Wasserstoff Halogen, Cyano oder für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylresten steht,
R² für Wasserstoff, Amino, Cyano oder für jeweils gegebenenfalls durch Halogen, Cyano oder C₁-C₄-Alkoxy substituiertes Alkyl, Alkoxy, Alkylamino oder Dialkylamino mit jeweils 1 bis 6 Kohlenstoffatomen in den Alkylresten steht, und
R³ für Wasserstoff, Cyano oder für jeweils gegebenenfalls durch Halogen, Cyano oder C₁-C₄-Alkoxy substituiertes Alkyl, Alkenyl oder Alkinyl mit jeweils bis zu 6 Kohlenstoffatomen oder für jeweils gegebenenfalls durch Halogen, Cyano, Carboxy, C₁-C₄-Alkyl oder C₁-C₄-Alkoxy-carbonyl substituiertes Cycloalkyl oder Cycloalkylalkyl mit 3 bis 6 Kohlenstoffatomen in den Cycloalkylteilen und gegebenenfalls 1 bis 4 Kohlenstoffatomen im Alkylteil steht,
G für Sauerstoff, für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- oder -N(R⁵)-CQ-Q-CH₂- steht,
wobei
n für die Zahlen 0, 1 oder 2 steht,
Q für Sauerstoff oder Schwefel steht,
R⁴ für Wasserstoff, Cyano, für gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
R⁵ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
Z für gegebenenfalls durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) substituiertes Alkyl mit 1 bis 8 Kohlenstoffatomen, für jeweils gegebenenfalls durch Halogen substituiertes Alkenyl oder Alkinyl mit jeweils bis zu 8 Kohlenstoffatomen, für jeweils gegebenenfalls durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen, für jeweils gegebenenfalls substituiertes Phenyl, Naphthyl oder für Heterocyclyl mit 3 bis 7 Ringgliedern, von denen mindestens eines für Sauerstoff Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, Cycloalkyl mit 3 bis 6 Kohlenstoffatomen, Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -, sowie jeweils gegebenenfalls im Phenylteil einfach oder mehrfach, gleich oder verschieden durch Halogen, Cyano und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen substituiertes Phenyl, Phenoxy, Benzyl, Benzyloxy, Phenylethyl oder Phenylethyloxy.

3. Verbindungen der Formel (I) gemäß Anspruch 1, in welcher
A für Wasserstoff, für gegebenenfalls durch Fluor, Chlor, Methoxy, Ethoxy, n- oder i-Propoxy, Methoxy-ethoxy, Ethoxy-ethoxy, Dimethylamino, Diethylamino, Acetylamino, Propionylamino, N-Methyl-acetylamino, N-Ethyl-acetylamino, N-Methyl-propionylamino, N-Ethyl-propionylamino, Methoxycarbonylamino, Ethoxycarbonylamino, N-Methyl-N-methoxycarbonylamino, N-Ethyl-N-methoxycarbonylamino, N-Methyl-N-ethoxycarbonylamino oder N-Ethyl-N-ethoxycarbonylamino substituiertes Methyl, Ethyl, n- oder i-Propyl, n-, i-, s- oder t-Butyl, für Methoxy-carbonyl, Ethoxycarbonyl, Methylaminocarbonyl, Ethylaminocarbonyl, Dimethylaminocarbonyl oder Diethylaminocarbonyl steht
A¹ für Wasserstoff, Methyl, Ethyl, n- oder i-Propyl, n-, i- oder s-Butyl steht,
Ar für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Trifluormethyl, Methoxy, Ethoxy, Methylthio, Methylsulfinyl oder Methylsulfonyl,
E für eine der nachstehenden Gruppierungen steht 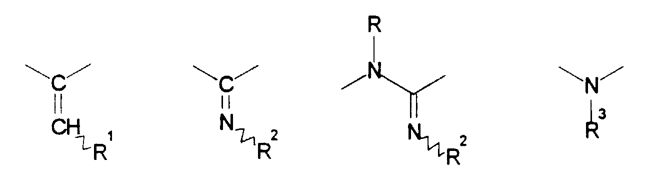 worin
R für Methyl, Ethyl, n- oder i-Propyl, n-, i- oder s-Butyl steht,
R¹ für Wasserstoff, Fluor, Chlor, Brom, Cyano oder für jeweils gegebenenfalls durch Fluor, Chlor, Cyano, Methoxy oder Ethoxy substituiertes Methyl, Ethyl, Propyl, Methoxy, Ethoxy, Methylthio, Ethylthio, Methylamino, Ethylamino oder Dimethylamino steht,
R² für Wasserstoff, Amino, Cyano oder für jeweils gegebenenfalls durch Fluor, Chlor, Cyano, Methoxy oder Ethoxy substituiertes Methyl, Ethyl, Methoxy, Ethoxy, Methylamino, Ethylamino oder Dimethylamino steht, und
R³ für Wasserstoff, Cyano oder für jeweils gegebenenfalls durch Fluor, Cyano, Methoxy oder Ethoxy substituiertes Methyl, Ethyl n- oder i-Propyl, n-, i- oder s-Butyl, für Allyl oder Propargyl oder für jeweils gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
G für Sauerstoff, für jeweils gegebenenfalls durch Fluor, Chlor, Hydroxy, Methyl, Ethyl, n- oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- oder -N(R⁵)-CQ-Q-CH₂- teht,
wobei
n für die Zahlen 0, 1 oder 2 steht,
Q für Sauerstoff oder Schwefel steht,
R⁴ für Wasserstoff, Cyano, für gegebenenfalls durch Fluor, Chlor, Cyano, Methoxy oder Ethoxy substituiertes Methyl, Ethyl, n- oder i-Propyl, n-, i- oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und
R⁵ für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht, und
Z für gegebenenfalls durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl, für jeweils gegebenenfalls durch Fluor, Chlor oder Brom substituiertes Allyl, Crotonyl, 1-Methyl-allyl, Propargyl oder 1-Methyl-propargyl , für jeweils gegebenenfalls durch Fluor, Chlor, Brom, Cyano, Carboxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substiuiertes Cyclopropyl, Cyclobutyl, Cyclopentyl oder Cyclohexyl, für jeweils gegebenenfalls substituiertes Phenyl, Naphthyl, Furyl, Thienyl, Oxazolyl, Isoxazolyl, Thiazolyl, Isothiazolyl, Oxadiazolyl, Thiadiazolyl, Pyridinyl, Pyrimidinyl, Pyridazinyl, Pyrazinyl, 1,2,3-Triazinyl, 1,2,4-Triazinyl, 1,3,5-Triazinyl, Oxiranyl, Oxetanyl, Tetrahydrofuryl, Perhydropyranyl. Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Difluormethoxy, Trifluormethoxy, Difluormethylthio, Trifluormethylthio, Trifluormethylsulfinyl oder Trifluormethylsulfonyl , Methylamino, Ethylamino, n- oder i-Propylamino, Dimethylamino, Diethylamino, Acetyl, Propionyl, Acetyloxy, Methoxy-carbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Ethyl, n- oder i-Propyl substituiertes Trimethylen (Propan-1,3-diyl), Methylendioxy oder Ethylendioxy, Cyclopropyl, Cyclobutyl, Cyclopentyl oder Cyclohexyl, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy.

4. Verbindungen der Formel (I) gemäß Anspruch 1, in welcher
A für Wasserstoff steht,
A¹ für Wasserstoff oder Methyl steht,
Ar für ortho-Phenylen, Pyridin-2,3-diyl oder Thiophen-2,3-diyl steht,
E für eine der nachstehenden Gruppierungen steht 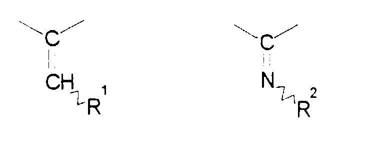 worin
R¹ und R² jeweils für Methoxy stehen,
G für Sauerstoff, Methylen oder eine der nachstehenden Gruppierungen -CH₂-O-, -O-CH₂-, -S(O)ₙ-, -CH₂-S(O)ₙ-, -S(O)ₙ-CH₂-, -C(R⁴)=N-O--O-N=C(R⁴)-, -C(R⁴)=N-O-CH₂-, -N(R⁵)- oder -CH₂-O-N=C(R⁴)- steht,
wobei
n für die Zahlen 0, 1 oder 2 steht,
R⁴ für Wasserstoff, Methyl oder Ethyl steht und
R⁵ für Wasserstoff, Methyl oder Ethyl steht, und
Z für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy

5. Schädlingsbekämpfungsmittel, gekennzeichnet durch einen Gehalt an mindestens einer Verbindung der Formel (I) nach Anspruch 1.

6. Verfahren zur Bekämpfung von Schädlingen, dadurch gekennzeichnet, daß man Verbindungen der Formel (I) nach Anspruch 1 auf Schädlinge und/oder ihren Lebensraum einwirken läßt.

7. Verwendung von Verbindungen der Formel (I) nach den Ansprüchen 1 bis 4 zur Bekämpfung von Schädlingen.

8. Verfahren zur Herstellung von Schädlingsbekämpfungsmitteln, dadurch gekennzeichnet, daß man Verbindungen der Formel (I) nach den Ansprüchen 1 bis 4 mit Streckmitteln und/oder oberflächenaktiven Mitteln vermischt.

9. Verfahren zur Herstellung von Hydroxamsäurederivaten der allgemeinen Formel (I) nach Anspruch 1, dadurch gekennzeichnet, daß man Carbonsäurederivate der allgemeinen Formel (II) 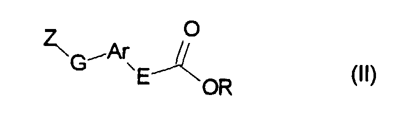 in welcher
Ar, E, G und Z die in Anspruch 1 angegebene Bedeutung haben und
R für Wasserstoff oder Alkyl steht,
mit einem Hydroxylamin der allgemeinen Formel (III)
A¹-NH-O-A (III)
in welcher
A und A¹ die in Anspruch 1 angegebene Bedeutung haben,
- oder mit einem Hydrogenhalogenid hiervon -
gegebenenfalls in Gegenwart eines Säureakzeptors und gegebenenfalls in Gegenwart eines Verdünnungsmittels umsetzt .

## Claims

1. Hydroxamic acid derivatives of the general formula (I), 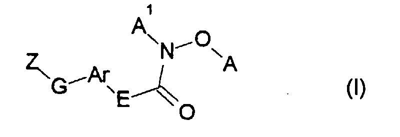 in which
A represents hydrogen, alkyl having 1 to 8 carbon atoms which is optionally substituted by halogen, cyano, hydroxyl, C₁-C₄-alkoxy, C₁-C₄-alkoxy-C₁-C₄-alkoxy, N,N-di-(C₁-C₄-alkyl)-amino, N- (C₁-C₄-alkyl-carbonyl)-amino, N-(C₁-C₄-alkyl)-N-(C₁-C₄-alkyl-carbonyl)-amino, N-(C₁-C₄-alkoxy-carbonyl)-amino or N-(C₁-C₄-alkyl)-N-(C₁-C₄-alkoxy-carbonyl)-amino, C₁-C₄-alkoxy-carbonyl C₁-C₄-alkylamino-carbonyl or di-(C₁-C₄-alkyl)-amino-carbonyl,
A¹ represents hydrogen or alkyl,
Ar represents in each case optionally substituted arylene or heteroarylene,
E represents a 1-alkene-1,1-diyl grouping which contains a radical R¹ in the 2 position, or represents a 2-aza-1-alkene-1,1-diyl grouping which contains a radical R² in the 2 position, or represents an optionally substituted imino grouping ("azamethylene", N-R³),
where
R¹ represents hydrogen, halogen or cyano, or in each case optionally substituted alkyl, alkoxy, alkylthio, alkylamino or dialkylamino,
R² represents hydrogen, amino or cyano, or in each case optionally substituted alkyl, alkoxy, alkylamino or dialkylamino, and
R³ represents hydrogen or cyano, or in each case optionally substituted alkyl, alkenyl, alkinyl, cycloalkyl or cycloalkylalkyl,
G represents oxygen, or alkanediyl, alkenediyl or alkinediyl which are in each case optionally substituted by halogen, hydroxyl, alkyl, halogenoalkyl or cycloalkyl, or one of the following groupings
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O=N-C(R⁴)-, -N(R⁵)-CQ-Q-, -CQ-N(R⁵)-CQ-Q-, or -N(R⁵)-CQ-Q-CH₂-,
where
n represents the numbers 0, 1 or 2,
Q represents oxygen or sulphur,
R⁴ represents hydrogen or cyano, or in each case optionally substituted alkyl, alkoxy, alkylthio, alkylamino, dialkylamino, or cycloalkyl, and
R⁵ represents hydrogen, hydroxyl or cyano, or in each case optionally substituted alkyl, alkoxy or cycloalkyl, and
Z represents in each case optionally substituted alkyl, alkenyl, alkinyl, cycloalkyl, aryl or heterocyclyl.

2. Compounds of the formula (I) according to Claim 1, in which
A represents hydrogen, alkyl having 1 to 8 carbon atoms which is optionally substituted by halogen, cyano, hydroxyl, C₁-C₄-alkoxy, C₁-C₄-alkoxy-C₁-C₄-alkoxy, N,N-di-(C₁-C₄-alkyl)-amino, N-(C₁-C₄-alkyl-carbonyl)-amino, N-(C₁-C₄-alkyl)-N-(C₁-C₄-alkyl-carbonyl)-amino, N-(C₁-C₄-alkoxy-carbonyl)-amino or N-(C₁-C₄-alkyl)-N-(C₁-C₄-alkoxy-carbonyl)-amino, C₁-C₄-alkoxy-carbonyl C₁-C₄-alkylamino-carbonyl or di-(C₁-C₄-alkyl)-amino-carbonyl,
A¹ represents hydrogen or alkyl having 1 to 6 carbon atoms,
Ar represents in each case optionally substituted phenylene or naphthylene, or heteroarylene having 5 or 6 ring members, of which at least one represents oxygen, sulphur or nitrogen and optionally one or two additional members represent nitrogen, where the possible substituents are preferably selected from the following list:
halogen, cyano, nitro, amino, hydroxyl, formyl, carboxyl, carbamoyl, thiocarbamoyl, in each case straight-chain or branched alkyl, alkoxy, alkylthio, alkylsulphinyl or alkylsulphonyl having in each case 1 to 6 carbon atoms, in each case straight-chain or branched alkenyl, alkenyloxy or alkinyloxy having in each case 2 to 6 carbon atoms, in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms, in each case straight-chain or branched halogenoalkenyl or halogenoalkenyloxy having in each case 2 to 6 carbon atoms and 1 to 13 identical or different halogen atoms, in each case straight-chain or branched alkylamino, dialkylamino, alkylcarbonyl, alkylcarbonyloxy, alkoxycarbonyl, alkylsulphonyloxy, hydroxyiminoalkyl or alkoxyiminoalkyl having in each case 1 to 6 carbon atoms in the individual alkyl moieties, or in each case doubly linked alkylene or dioxyalkylene which have in each case 1 to 6 carbon atoms and which are in each case optionally substituted, once or more than once, identically or differently, by halogen and/or straight-chain or branched alkyl having 1 to 4 carbon atoms and/or straight-chain or branched halogenoalkyl having 1 to 4 carbon atoms and 1 to 9 identical or different halogen atoms,
E represents one of the following groupings 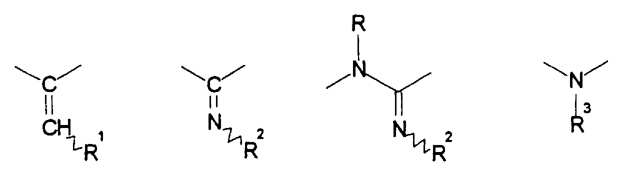 in which
R represents hydrogen or alkyl having 1 to 6 carbon atoms,
R¹ represents hydrogen, halogen or cyano, or alkyl, alkoxy, alkylthio, alkylamino or dialkylamino which have in each case 1 to 6 carbon atoms in the alkyl radicals and which are in each case optionally substituted by halogen, cyano or C₁-C₄-alkoxy,
R² represents hydrogen, amino or cyano, or alkyl, alkoxy, alkylamino or dialkylamino which have in each case 1 to 6 carbon atoms in the alkyl radicals and which are in each case optionally substituted by halogen, cyano or C₁-C₄-alkoxy, and
R³ represents hydrogen or cyano, or alkyl, alkenyl or alkinyl which have in each case up to 6 carbon atoms and which are in each case optionally substituted by halogen, cyano or C₁-C₄-alkoxy, or cycloalkyl or cycloalkylalkyl which have 3 to 6 carbon atoms in the cycloalkyl moieties and optionally 1 to 4 carbon atoms in the alkyl moiety and which are in each case optionally substituted by halogen, cyano, carboxyl, C₁-C₄-alkyl or C₁-C₄-alkoxy-carbonyl,
G represents oxygen, alkanediyl, alkenediyl or alkinediyl which have in each case up to 4 carbon atoms and which are in each case optionally substituted by halogen, hydroxyl, C₁-C₄-alkyl, C₁-C₄-halogenoalkyl or C₃-C₆-cycloalkyl, or one of the following groupings
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, or -N(R⁵)-CQ-Q-CH₂-,
where
n represents the numbers 0, 1 or 2,
Q represents oxygen or sulphur,
R⁴ represents hydrogen or cyano, or alkyl, alkoxy, alkylthio, alkylamino, or dialkylamino which have in each case 1 to 6 carbon atoms in the alkyl groups and which are optionally substituted by halogen, cyano or C₁-C₄-alkoxy, or cycloalkyl which has from 3 to 6 carbon atoms and which is in each case optionally substituted by halogen, cyano, carboxyl, C₁-C₄-alkyl or C₁-C₄-alkoxy-carbonyl, and
R⁵ represents hydrogen, hydroxyl or cyano, or alkyl which has 1 to 6 carbon atoms and which is optionally substituted by halogen, cyano or C₁-C₄-alkoxy, or cycloalkyl which has 3 to 6 carbon atoms and which is optionally substituted by halogen, cyano, carboxyl, C₁-C₄-alkyl or C₁-C₄-alkoxy-carbonyl, and
Z represents alkyl which has 1 to 8 carbon atoms and which is optionally substituted by halogen, cyano, hydroxyl, amino, C₁-C₄-alkoxy, C₁-C₄-alkylthio, C₁-C₄-alkylsulphinyl or C₁-C₄-alkylsulphonyl (which are in each case optionally substituted by halogen), alkenyl or alkinyl which have in each case up to 8 carbon atoms and which are in each case optionally substituted by halogen, cycloalkyl which has 3 to 6 carbon atoms and which is in each case optionally substituted by halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl or C₁-C₄-alkoxycarbonyl, in each case optionally substituted phenyl or naphthyl, or heterocyclyl having 3 to 7 ring members, of which at least one represents oxygen, sulphur or nitrogen and optionally one or two additional members represent nitrogen, where the possible substituents are preferably selected from the following list:
halogen, cyano, nitro, amino, hydroxyl, formyl, carboxyl, carbamoyl, thiocarbamoyl, in each case straight-chain or branched alkyl, alkoxy, alkylthio, alkylsulphinyl or alkylsulphonyl having in each case 1 to 6 carbon atoms, in each case straight-chain or branched alkenyl or alkenyloxy having in each case 2 to 6 carbon atoms, in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms, in each case straight-chain or branched halogenoalkenyl or halogenoalkenyloxy having in each case 2 to 6 carbon atoms and 1 to 13 identical or different halogen atoms, in each case straight-chain or branched alkylamino, dialkylamino, alkylcarbonyl, alkylcarbonyloxy, alkoxycarbonyl, alkylsulphonyloxy, hydroxyiminoalkyl or alkoxyiminoalkyl having in each case 1 to 6 carbon atoms in the individual alkyl moieties, in each case doubly linked alkylene or dioxyalkylene which have in each case 1 to 6 carbon atoms and which are in each case optionally substituted, once or more than once, identically or differently, by halogen and/or straight-chain or branched alkyl having 1 to 4 carbon atoms and/or straight-chain or branched halogenoalkyl having 1 to 4 carbon atoms and 1 to 9 identical or different halogen atoms, cycloalkyl having 3 to 6 carbon atoms, heterocyclyl or heterocyclyl-methyl having in each case 3 to 7 ring members, of which in each case 1 to 3 are identical or different heteroatoms - in particular nitrogen, oxygen and/or sulphur -, and also phenyl, phenoxy, benzyl, benzyloxy, phenylethyl or phenylethyloxy which are in each case optionally substituted in the phenyl moiety, once or more than once, identically or differently, by halogen, cyano and/or straight-chain or branched alkyl having 1 to 4 carbon atoms and/or straight-chain or branched halogenoalkyl having 1 to 4 carbon atoms and 1 to 9 identical or different halogen atoms and/or straight-chain or branched alkoxy having 1 to 4 carbon atoms and/or straight-chain or branched halogenoalkoxy having 1 to 4 carbon atoms and 1 to 9 identical or different halogen atoms.

3. Compounds of the formula (I) according to Claim 1, in which
A represents hydrogen, methyl, ethyl, n- or i-propyl, or n-, i-, s- or t-butyl which are optionally substituted by fluorine, chlorine, methoxy, ethoxy, n- or i-propoxy, methoxyethoxy, ethoxy-ethoxy, dimethylamino, diethylamino, acetylamino, propionylamino, N-methyl-acetylamino, N-ethyl-acetylamino, N-methyl-propionylamino, N-ethyl-propionylamino, methoxycarbonylamino, ethoxycarbonylamino, N-methyl-N-methoxycarbonylamino, N-ethyl-N-methoxycarbonylamino, N-methyl-N-ethoxycarbonylamino or N-ethyl-N-ethoxycarbonylamino, methoxycarbonyl, ethoxycarbonyl, methylaminocarbonyl, ethylaminocarbonyl, dimethylaminocarbonyl or diethylaminocarbonyl,
A¹ represents hydrogen, methyl, ethyl, n- or i-propyl, or n-, i- or s-butyl,
Ar represents in each case optionally substituted ortho-, meta- or para-phenylene, furandiyl, thiophenediyl, pyrrolediyl, pyrazolediyl, triazolediyl, oxazolediyl, isoxazolediyl, thiazolediyl, isothiazolediyl, oxadiazolediyl, thiadiazolediyl, pyridinediyl (in particular pyridine-2,3-diyl), pyrimidinediyl, pyridazinediyl, pyrazinediyl, 1,3,4-triazinediyl or 1,2,3,-triazinediyl, where the possible substituents are selected, in particular, from the following list:
fluorine, chlorine, cyano, methyl, ethyl, trifluoromethyl, methoxy, ethoxy, methylthio, methylsulphinyl or methylsulphonyl,
E represents one of the following groupings 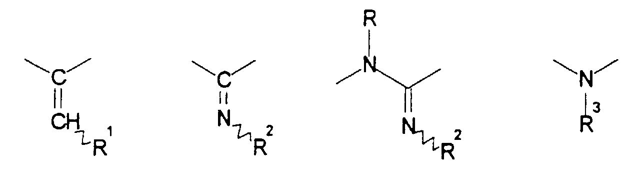 in which
R represents methyl, ethyl, n- or i-propyl, or n-, i- or s-butyl,
R¹ represents hydrogen, fluorine, chlorine, bromine or cyano, or methyl, ethyl, propyl, methoxy, ethoxy, methylthio, ethylthio, methylamino, ethylamino or dimethylamino which are in each case optionally substituted by fluorine, chlorine, cyano, methoxy or ethoxy,
R² represents hydrogen, amino or cyano, or methyl, ethyl, methoxy, ethoxy, methylamino, ethylamino or dimethylamino which are in each case optionally substituted by fluorine, chlorine, cyano, methoxy or ethoxy, and
R³ represents hydrogen or cyano, or methyl, ethyl, n- or i-propyl, or n-, i- or s-butyl which are in each case optionally substituted by fluorine, cyano, methoxy or ethoxy, represents allyl or propargyl, or represents cyclopropyl, cyclobutyl, cyclopentyl, cyclohexyl, cyclopropylmethyl, cyclobutylmethyl, cyclopentylmethyl or cyclohexylmethyl which are in each case optionally substituted by fluorine, chlorine, cyano, carboxyl, methyl, ethyl, n- or i-propyl, methoxy-carbonyl or ethoxy-carbonyl,
G represents oxygen, methylene, dimethylene (ethane-1,2-diyl), ethene-1,2-diyl, ethine-1,2-diyl which are in each case optionally substituted by fluorine, chlorine, hydroxyl, methyl, ethyl, n- or i-propyl, trifluoromethyl, cyclopropyl, cyclobutyl, cyclopentyl or cyclohexyl, or one of the following groupings
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N (R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, or -N(R⁵)-CQ-Q-CH₂-,
where
n represents the numbers 0, 1 or 2,
Q represents oxygen or sulphur,
R⁴ represents hydrogen or cyano, or methyl, ethyl, n- or i-propyl, n-, i- or s-butyl, methoxy, ethoxy, propoxy, butoxy, methylthio, ethylthio, propylthio, butylthio, methylamino, ethylamino, propylamino, dimethylamino or diethylamino which are optionally substituted by fluorine, chlorine, cyano, methoxy or ethoxy, or cyclopropyl, cyclobutyl, cyclopentyl or cyclohexyl which are in each case optionally substituted by fluorine, chlorine, cyano, carboxyl, methyl, ethyl, n- or i-propyl, methoxycarbonyl or ethoxy-carbonyl, and
R⁵ represents hydrogen, hydroxyl or cyano, or methyl, ethyl, n- or i-propyl, or n-, i-, s- or t-butyl which are in each case optionally substituted by fluorine, chlorine, cyano, methoxy or ethoxy, or cyclopropyl, cyclobutyl, cyclopentyl or cyclohexyl which are optionally substituted by fluorine, chlorine, cyano, carboxyl, methyl, ethyl, n- or i-propyl, methoxy-carbonyl or ethoxy-carbonyl, and
Z represents methyl, ethyl, n- or i-propyl, or n-, i-, s- or t-butyl which are optionally substituted by fluorine, chlorine, bromine, cyano, hydroxyl, amino, methoxy, ethoxy, methylthio, ethylthio, methylsulphinyl, ethylsulphinyl, methylsulphonyl or ethylsulphonyl (which are in each case optionally substituted by fluorine and/or chlorine), allyl, crotonyl, 1-methyl-allyl, propargyl or 1-methyl-propargyl which are in each case optionally substituted by fluorine, chlorine or bromine, cyclopropyl, cyclobutyl, cyclopentyl or cyclohexyl which are in each case optionally substituted by fluorine, chlorine, bromine, cyano, carboxyl, phenyl (which is optionally substituted by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy), methyl, ethyl, n- or i-propyl, methoxy-carbonyl or ethoxy-carbonyl, or in each case optionally substituted phenyl, naphthyl, furyl, thienyl, oxazolyl, isoxazolyl, thiazolyl, isothiazolyl, oxadiazolyl, thiadiazolyl, pyridinyl, pyrimidinyl, pyridazinyl, pyrazinyl, 1,2,3-triazinyl, 1,2,4-triazinyl, 1,3,5-triazinyl, oxiranyl, oxetanyl, tetrahydrofuryl, perhydropyranyl, pyrrolidinyl, piperidinyl or morpholinyl, where the possible substituents are preferably selected from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluoromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroxyiminomethyl, hydroxyiminoethyl, methoxyiminomethyl, ethoxyiminomethyl, methoxyiminoethyl or ethoxyiminoethyl; trimethylene (propane-1,3-diyl), methylenedioxy, ethylenedioxy which are in each case optionally substituted, once or more than once, identically or differently, by fluorine, chlorine, methyl, ethyl, or n- or i-propyl, cyclopropyl, cyclobutyl, cyclopentyl or cyclohexyl, and also phenyl, phenoxy, benzyl or benzyloxy which are in each case optionally substituted in the phenyl moiety, once or more than once, identically or differently, by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy.

4. Compounds of the formula (I) according to Claim 1, in which
A represents hydrogen,
A¹ represents hydrogen or methyl,
Ar represents ortho-phenylene, pyridine-2,3-diyl or thiophene-2,3-diyl,
E represents one of the following groupings 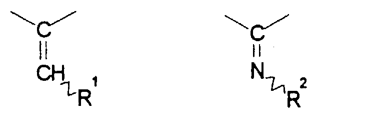 in which
R¹ and R² in each case represent methoxy,
G represents oxygen, methylene or one of the following groupings
-CH₂-O-, -O-CH₂-, -S(O)ₙ-, -CH₂-S(O)ₙ-, -S(O)ₙ-CH₂-, -C(R⁴)=N-O-, -O-N=C(R⁴)-, -C(R⁴)=N-O-CH₂-, -N(R⁵)- or -CH₂-O-N=C(R⁴)-,
where
n represents the numbers 0, 1 or 2,
R⁴ represents hydrogen, methyl or ethyl, and
R⁵ represents hydrogen, methyl or ethyl, and
Z represents in each case optionally substituted phenyl, pyridinyl, pyrimidinyl, pyridazinyl, pyrazinyl, 1,2,3-triazinyl, 1,2,4-triazinyl or 1,3,5-triazinyl, where the possible substituents are preferably selected from the following list:
fluorine, chlorine, bromine, cyano, methyl, ethyl, n- or i-propyl, n-, i-, s- or t-butyl, methoxy, ethoxy, n- or i-propoxy, methylthio, ethylthio, nor i-propylthio, methylsulphinyl, ethylsulphinyl, methylsulphonyl or ethylsulphonyl, trifluoromethyl, difluoromethoxy, trifluoromethoxy, difluoromethylthio, trifluoromethylthio, trifluoromethylsulphinyl or trifluoromethylsulphonyl, methoxycarbonyl, ethoxycarbonyl, methoxyiminomethyl, ethoxyiminomethyl, methoxyiminoethyl or ethoxyiminoethyl, or methylenedioxy or ethylenedioxy which are in each case optionally substituted, once or more than once, identically or differently, by fluorine, chlorine, methyl or ethyl, and also phenyl, phenoxy, benzyl or benzyloxy which are in each case optionally substituted in the phenyl moiety, once or more than once, identically or differently, by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy.

5. Pesticide, characterized by a content of at least one compound of the formula (I) according to Claim 1.

6. Process for controlling pests, characterized in that compounds of the formula (I) according to Claim 1 are permitted to act on pests and/or their habitat.

7. Use of compounds of the formula (I) according to Claims 1 to 4 for controlling pests.

8. Process for producing pesticides, characterized in that compounds of the formula (I) according to Claims 1 to 4 are mixed with extenders and/or surface-active agents.

9. Process for preparing hydroxamic acid derivatives of the general formula (I) according to Claim 1, characterized in that carboxylic acid derivatives of the general formula (II) 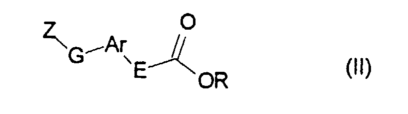 in which
Ar, E, G and Z have the meaning stated in Claim 1, and
R represents hydrogen or alkyl,
are reacted with a hydroxylamine of the general formula (III)
A¹-NH-O-A (III)
in which
A and A¹ have the meaning stated in Claim 1,
- or with a hydrogen halide thereof -
optionally in the presence of an acid acceptor and optionally in the presence of a diluent.

## Revendications

1. Dérivés d'acide hydroxamique de formule générale (I) 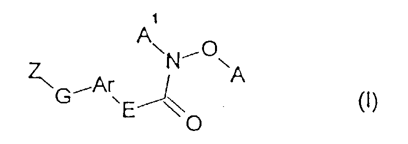 dans laquelle
A représente l'hydrogène, un groupe alkyle ayant 1 à 8 atomes de carbone portant éventuellement un substituant halogéno, cyano, hydroxy, alkoxy en C₁ à C₄, (alkoxy en C₁ à C₄)-(alkoxy en C₁ à C₄), N,N-di-(alkyle en C₁ à C₄)-amino, N-[(alkyle en C₁ à C₄)-carbonyl]-amino, N-(alkyle en C₁ à C₄)-N-[(alkyle en C₁ à C₄)-carbonyl]-amino, N-[(alkoxy en C₁ à C₄)-carbonyl]-amino ou N-(alkyle en C₁ à C₄)-N-[(alkoxy en C₁ à C₄)-carbonyl]-amino, un groupe (alkoxy en C₁ à C₄)-carbonyle, (alkyle en C₁ à C₄)-aminocarbonyle ou di-(alkyle en C₁ à C₄)-aminocarbonyle,
A¹ représente l'hydrogène ou un groupe alkyle,
Ar représente un groupe arylène ou hétéroarylène dont chacun est éventuellement substitué,
E est un groupement 1-alcène-1,1-diyle qui porte un reste R¹ en position 2 ou représente un groupement 2-aza-1-alcène-1,1-diyle qui porte un reste R² en position 2, ou un groupement imino éventuellement substitué ("azaméthylène", N-R³), où
R¹ représente l'hydrogène, un halogène, un groupe cyano ou un groupe, éventuellement substitué dans chaque cas, alkyle, alkoxy, alkylthio, alkylamino ou dialkylamino,
R² représente l'hydrogène, un groupe amino, cyano ou un groupe, éventuellement substitué dans chaque cas, alkyle, alkoxy, alkylamino ou dialkylamino, et
R³ représente l'hydrogène, un groupe cyano ou un groupe, éventuellement substitué dans chaque cas, alkyle, alcényle, alcynyle, cycloalkyle ou cycloalkylalkyle,
G représente l'oxygène, un groupe alcanediyle, alcènediyle, alcynediyle dont chacun porte éventuellement un substituant halogéno, hydroxy, alkyle, halogénalkyle ou cycloalkyle, ou l'un des groupements suivants
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, C(R⁴)=N-O-CH₂-, -N(R⁵)-, -CQ-N(R⁵)-, -N(R⁵)-CQ-, -Q-CQ-N(R⁵)-, N=C(R⁴)-Q-CH₂-, CH₂-O=N-C(R⁴)-, -N(R⁵)-CQ-Q-, CQ-N(R⁵)-CQ-Q- ou -N(R⁵)-CQ-Q-CH₂-
où
n représente les nombres 0, 1 ou 2,
Q est l'oxygène ou le soufre,
R⁴ est l'hydrogène, un groupe cyano ou un groupe, éventuellement substitué dans chaque cas, alkyle, alkoxy, alkylthio, alkylamino, dialkylamino ou cycloalkyle et
R⁵ représente l'hydrogène, un groupe hydroxy, cyano ou un groupe alkyle, alkoxy ou cycloalkyle éventuellement substitué dans chaque cas, et
Z représente un groupe alkyle, alcényle, alcynyle, cycloalkyle, aryle ou hétérocyclyle éventuellement substitué dans chaque cas.

2. Composés de formule (I) suivant la revendication 1, dans laquelle
A représente l'hydrogène, un groupe alkyle ayant 1 à 8 atomes de carbone portant éventuellement un substituant halogéno, cyano, hydroxy, alkoxy en C₁ à C₄, (alkoxy en C₁ à C₄)-(alkoxy en C₁ à C₄), N,N-di-(alkyle en C₁ à C₄)-amino, N-[(alkyle en C₁ à C₄)-carbonyl]-amino, N-(alkyle en C₁ à C₄)-N-[(alkyle en C₁ à C₄)-carbonyl]-amino, N-[(alkoxy en C₁ à C₄)-carbonyl]-amino ou N-(alkyle en C₁ à C₄)-N-[(alkoxy en C₁ à C₄)-carbonyl]-amino, un groupe (alkoxy en C₁ à C₄)-carbonyle, (alkyle en C₁ à C₄)-aminocarbonyle ou di-(alkyle en C₁ à C₄)-aminocarbonyle,
A¹ représente l'hydrogène ou un groupe alkyle ayant 1 à 6 atomes de carbone,
Ar représente un groupe phénylène ou naphtylène dont chacun est éventuellement substitué ou un groupe hétéroarylène à 5 ou 6 chaînons dont l'un au moins est l'oxygène, le soufre ou l'azote et le cas échéant un autre ou deux autres représentent de l'azote, les substituants possibles étant choisis de préférence parmi ceux qui sont énumérés ci-après :
halogène, cyano, nitro, amino, hydroxy, formyle, carboxy, carbamoyle, thiocarbamoyle ; alkyle, alkoxy, alkylthio, alkylsulfinyle ou alkylsulfonyle ayant chacun 1 à 6 atomes de carbone et étant chacun linéaire ou ramifié ; alcényle, alcényloxy ou alcynyloxy ayant chacun 2 à 6 atomes de carbone et étant chacun linéaire ou ramifié ; halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et étant chacun linéaire ou ramifié ; halogénalcényle ou halogénalcényloxy ayant chacun 2 à 6 atomes de carbone et 1 à 13 atomes d'halogènes identiques ou différents et étant chacun linéaire ou ramifié ; alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et étant chacun linéaire ou ramifié ; alkylène ou dioxyalkylène ayant chacun 1 à 6 atomes de carbone et chacun deux liaisons et portant chacun le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents,
E représente l'un des groupements suivants 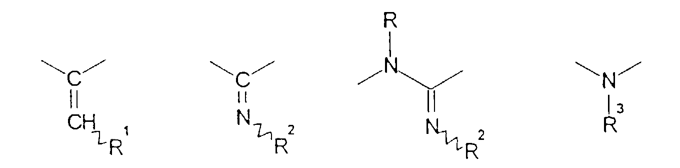 où
R représente l'hydrogène ou un groupe alkyle ayant 1 à 6 atomes de carbone,
R¹ est l'hydrogène, un halogène, un groupe cyano ou un groupe alkyle, alkoxy, alkylthio, alkylamino ou dialkylamino ayant chacun 1 à 6 atomes de carbone dans les restes alkyle et portant chacun le cas échéant un substituant halogéno, cyano ou alkoxy en C₁ à C₄,
R² représente l'hydrogène, un groupe amino, cyano ou un groupe alkyle, alkoxy, alkylamino ou dialkylamino ayant chacun 1 à 6 atomes de carbone dans les restes alkyle et portant chacun le cas échéant un substituant halogéno, cyano ou alkoxy en C₁ à C₄, et
R³ représente l'hydrogène, un groupe cyano ou un groupe alkyle, alcényle ou alcynyle ayant chacun jusqu'à 6 atomes de carbone et portant chacun le cas échéant un substituant halogéno, cyano ou alkoxy en C₁ à C₄, ou un groupe cycloalkyle ou cycloalkylalkyle ayant 3 à 6 atomes de carbone dans les parties cycloalkyle et le cas échéant 1 à 4 atomes de carbone dans la partie alkyle et portant chacun le cas échéant un substituant halogéno, cyano, carboxy, alkyle en C₁ à C₄ ou (alkoxy en C₁ à C₄)-carbonyle,
G représente l'oxygène, un groupe alcanediyle, alcènediyle, alcynediyle ayant chacun jusqu'à 4 atomes de carbone et portant chacun le cas échéant un substituant halogéno, hydroxy, alkyle en C₁ à C₄, halogénalkyle en C₁ à C₄, cycloalkyle en C₃ à C₆, ou l'un des groupements suivants :
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-,-N=N-, -S(O)ₙ-, -CH₂-S(O)ₙ-, -CQ-, -S(O)ₙ-CH₂-,-C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- ou -N(R⁵)-CQ-Q-CH₂-
où
n représente les nombres 0, 1 ou 2,
Q représente l'oxygène ou le soufre,
R⁴ est l'hydrogène, un groupe cyano, un groupe alkyle, alkoxy, alkylthio, alkylamino ou dialkylamino ayant chacun 1 à 6 atomes de carbone dans les groupes alkyle et portant chacun le cas échéant un substituant halogéno, cyano ou alkoxy en C₁ à C₄, ou un groupe cycloalkyle ayant 3 à 6 atomes de carbone et portant le cas échéant un substituant halogéno, cyano, carboxy, alkyle en C₁ à C₄ ou (alkoxy en C₁ à C₄)-carbonyle, et
R⁵ représente l'hydrogène, un groupe hydroxy, cyano ou un groupe alkyle ayant 1 à 6 atomes de carbone et portant le cas échéant un substituant halogéno, cyano ou alkoxy en C₁ à C₄, ou un groupe cycloalkyle ayant 3 à 6 atomes de carbone éventuellement substitué par un radical halogéno, cyano, carboxy, alkyle en C₁ à C₄ ou (alkoxy en C₁ à C₄)-carbonyle, et
Z représente un groupe alkyle ayant 1 à 8 atomes de carbone éventuellement substitué par des radicaux halogéno, cyano, hydroxy, amino, alkoxy en C₁ à C₄, alkylthio en C₁ à C₄, alkylsulfinyle en C₁ à C₄ ou alkylsulfonyle en C₁ à C₄ (qui sont éventuellement substitués dans chaque cas par un halogène), un groupe alcényle ou alcynyle ayant chacun jusqu'à 8 atomes de carbone et portant chacun le cas échéant un substituant halogéno, un groupe cycloalkyle ayant 3 à 6 atomes de carbone portant le cas échéant un substituant halogéno, cyano, carboxy, phényle (qui est substitué le cas échéant par un halogène, un radical cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle, un groupe, éventuellement substitué dans chaque cas, phényle, naphtyle ou un groupe hétérocyclyle ayant 3 à 7 chaînons dont l'un au moins est l'oxygène, le soufre ou l'azote et, le cas échéant, un ou deux autres chaînons représentent de l'azote, les substituants possibles étant choisis de préférence parmi les substituants suivants :
halogène, cyano, nitro, amino, hydroxy, formyle, carboxy, carbamoyle, thiocarbamoyle ; alkyle, alkoxy, alkylthio, alkylsulfinyle ou alkylsulfonyle ayant chacun 1 à 6 atomes de carbone et étant chacun linéaire ou ramifié ; un groupe alcényle ou alcényloxy ayant chacun 2 à 6 atomes de carbone et étant chacun linéaire ou ramifié ; un groupe halogénalkyle, halogénalkoxy, halogénylalkylthio, halogénalkylsulfinyle ou halogénalkylsulfonyle ayant chacun 1 à 6 atomes de carbone et 1 à 13 atomes d'halogènes identiques ou différents et étant chacun linéaire ou ramifié ; un groupe halogénalcényle ou halogénalcényloxy ayant chacun 2 à 6 atomes de carbone et 1 à 13 atomes d'halogènes identiques ou différents et étant chacun linéaire ou ramifié ; un groupe alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et étant chacun linéaire ou ramifié ; un groupe alkylène ou dioxyalkylène ayant chacun 1 à 6 atomes de carbone, portant chacun deux liaisons et dont chacun est éventuellement substitué une ou plusieurs fois identiques ou différentes par un halogène et/ou un groupe alkyle linéaire ou ramifié ayant 1 à 4 atomes de carbone et/ou un groupe halogénalkyle linéaire ou ramifié ayant 1 à 4 atomes de carbone et 1 à 9 atomes d'halogènes identiques ou différents ; un groupe cycloalkyle ayant 3 à 6 atomes de carbone, hétérocyclyle ou hétérocyclyl-méthyle ayant dans chaque cas 3 à 7 chaînons, dont 1 à 3 sont dans chaque cas des hétéroatomes identiques ou différents - notamment azote, oxygène et/ou soufre -, ainsi qu'un groupe phényle, phénoxy, benzyle, benzyloxy, phényléthyle ou phényléthyloxy dont chacun est substitué le cas échéant dans la partie phényle une ou plusieurs fois identiques ou différentes par un halogène, un radical cyano et/ou un radical alkyle linéaire ou ramifié ayant 1 à 4 atomes de carbone et/ou un radical halogénalkyle linéaire ou ramifié ayant 1 à 4 atomes de carbone et 1 à 9 atomes d'halogènes identiques ou différents et/ou un radical alkoxy linéaire ou ramifié ayant 1 à 4 atomes de carbone et/ou un radical halogénalkoxy linéaire ou ramifié ayant 1 à 4 atomes de carbone et 1 à 9 atomes d'halogènes identiques ou différents.

3. Composés de formule (I) suivant la revendication 1, dans laquelle
A représente l'hydrogène, un groupe méthyle, éthyle, n-propyle, isopropyle, n-butyle, isobutyle, sec.-butyle, tertio-butyle portant le cas échéant un substituant fluoro, chloro, méthoxy, éthoxy, n-propoxy, isopropoxy, méthoxyéthoxy, éthoxy-éthoxy, diméthylamino, diéthylamino, acétylamino, propionylamino, N-méthylacétylamino, N-éthyl-acétylamino, N-méthylpropionylamino, N-éthyl-propionylamino, méthoxycarbonylamino, éthoxycarbonylamino, N-méthyl-N-méthoxycarbonylamino, N-éthyl-N-méthoxycarbonylamino, N-méthyl-N-éthoxycarbonylamino ou N-éthyl-N-éthoxycarbonylamino, un groupe méthoxycarbonyle, éthoxycarbonyle, méthylaminocarbonyle, éthylaminocarbonyle, diméthylaminocarbonyle ou diéthylaminocarbonyle,
A¹ représente l'hydrogène, un groupe méthyle, éthyle, n-propyle, isopropyle, n-butyle, isobutyle ou sec.-butyle,
Ar représente un groupe, éventuellement substitué dans chaque cas, ortho-, méta- ou para-phénylène,
un groupe furannediyle, thiophenediyle, pyrrolediyle, pyrazolediyle, triazolediyle, oxazolediyle, isoxazolediyle, thiazolediyle, isothiazolediyle, oxadiazolediyle, thiadiazolediyle, pyridinediyle, (notamment pyridine-2,3-diyle), pyrimidinediyle, pyridazinediyle, pyrazinediyle, 1,3,4-triazinediyle ou 1,2,3-triazinediyle, les substituants possibles étant choisis en particulier parmi ceux qui sont énumérés ci-après : fluor, chlore, cyano, méthyle, éthyle, trifluorométhyle, méthoxy, éthoxy, méthylthio, méthylsulfinyle ou méthylsulfonyle,
E représente l'un des groupements suivants 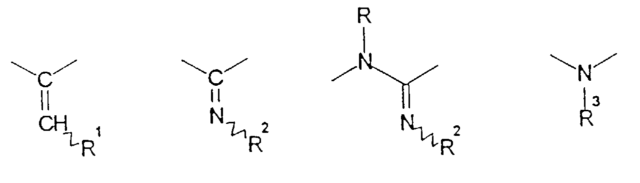 où
R est un groupe méthyle, éthyle, n-propyle, isopropyle, n-butyle, isobutyle ou sec.-butyle,
R¹ est l'hydrogène, le fluor, le chlore, le brome, un groupe cyano ou un groupe méthyle, éthyle, propyle, méthoxy, éthoxy, méthylthio, éthylthio, méthylamino, éthylamino ou diméthylamino, dont chacun est éventuellement substitué par du fluor, du chlore, un radical cyano, méthoxy ou éthoxy,
R² est l'hydrogène, un groupe amino, cyano ou un groupe méthyle, éthyle, méthoxy, éthoxy, méthylamino, éthylamino ou diméthylamino dont chacun est substitué le cas échéant par du fluor, du chlore, un radical cyano, méthoxy ou éthoxy, et
R³ représente l'hydrogène, un groupe cyano ou un groupe méthyle, éthyle, n-propyle, isopropyle, n-butyle, isobutyle ou sec.-butyle dont chacun est substitué le cas échéant par du fluor, un radical cyano, méthoxy ou éthoxy, un groupe allyle ou propargyle ou un groupe cyclopropyle, cyclobutyle, cyclopentyle, cyclohexyle, cyclopropylméthyle, cyclobutylméthyle, cyclopentylméthyle ou cyclohexylméthyle dont chacun est substitué le cas échéant par un radical fluoro, chloro, cyano, carboxy, méthyle, éthyle, n-propyle, isopropyle, méthoxycarbonyle ou éthoxycarbonyle,
G représente l'oxygène, un groupe méthylène, diméthylène(éthane-1,2-diyle),éthène-1,2-diyle, éthyne-1,2-diyle portant chacun le cas échéant un radical fluoro, chloro, hydroxy, méthyle, éthyle, n-propyle, isopropyle, trifluorométhyle, cyclopropyle, cyclobutyle, cyclopentyle ou cyclohexyle, ou l'un des groupements suivants :
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- ou -N(R⁵)-CQ-Q-CH₂-
où
n représente les nombres 0, 1 ou 2,
Q est l'oxygène ou le soufre,
R⁴ est l'hydrogène, un groupe cyano, un groupe, éventuellement substitué par un radical fluoro, chloro, cyano, méthoxy ou éthoxy, méthyle, éthyle, n-propyle, isopropyle, n-butyle, isobutyle, sec.-butyle, méthoxy, éthoxy, propoxy, butoxy, méthylthio, éthylthio, propylthio, butylthio, méthylamino, éthylamino, propylamino, diméthylamino ou diéthylamino, ou un groupe cyclopropyle, cyclobutyle, cyclopentyle ou cyclohexyle portant chacun le cas échéant un substituant fluoro, chloro, cyano, carboxy, méthyle, éthyle, n-propyle, isopropyle, méthoxycarbonyle ou éthoxycarbonyle, et
R⁵ est l'hydrogène, un groupe hydroxy, cyano, ou un groupe méthyle, éthyle, n-propyle, isopropyle, n-butyle, isobutyle, sec.-butyle, tertio-butyle portant chacun le cas échéant un substituant fluoro, chloro, cyano, méthoxy ou éthoxy, ou un groupe cyclopropyle, cyclobutyle, cyclopentyle ou cyclohexyle portant chacun le cas échéant un substituant fluoro, chloro, cyano, carboxy, méthyle, éthyle, n-propyle, isopropyle, méthoxycarbonyle ou éthoxycarbonyle, et
Z est un groupe méthyle, éthyle, n-propyle, isopropyle, n-butyle, isobutyle, sec.-butyle ou tertio-butyle portant le cas échéant un substituant fluoro, chloro, bromo, cyano, hydroxy, amino, méthoxy, éthoxy, méthylthio, éthylthio, méthylsulfinyle, éthylsulfinyle, méthylsulfonyle, éthylsulfonyle (substitués chacun le cas échéant par du fluor et/ou du chlore) ; un groupe allyle, crotonyle, 1-méthylallyle, propargyle ou 1-méthylpropargyle dont chacun est substitué le cas échéant par du fluor, du chlore ou du brome ; un groupe cyclopropyle, cyclobutyle, cyclopentyle ou cyclohexyle dont chacun est substitué le cas échéant par du fluor, du chlore, du brome, un radical cyano, carboxy, phényle, (qui est substitué le cas échéant par du fluor, du chlore, du brome, un radical cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy), méthyle, éthyle, n-propyle, isopropyle, méthoxycarbonyle ou éthoxycarbonyle ; un groupe phényle, naphtyle, furyle, thiényle, oxazolyle, isoxazolyle, thiazolyle, isothiazolyle, oxadiazolyle, thiadiazolyle, pyridinyle, pyrimidinyle, pyridazinyle, pyrazinyle, 1,2,3-triazinyle, 1,2,4-triazinyle, 1,3,5-triazinyle, oxirannyle, oxétannyle, tétrahydrofuryle, perhydropyrannyle, pyrrolidinyle, pipéridinyle ou morpholinyle dont chacun est éventuellement substitué, les substituants possibles étant choisis de préférence parmi les substituants énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle, éthoximinoéthyle ; triméthylène (propane-1,3-diyle), méthylènedioxy ou éthylènedioxy, cyclopropyle, cyclobutyle, cyclopentyle ou cyclohexyle dont chacun porte le cas échéant un ou plusieurs substituants, identiques ou différents, fluoro, chloro, méthyle, éthyle, n-propyle ou isopropyle ; ainsi que phényle, phénoxy, benzyle ou benzyloxy dont chacun porte le cas échéant dans la partie phényle un ou plusieurs substituants, identiques ou différents, fluoro, chloro, bromo, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy.

4. Composés de formule (I) suivant la revendication 1, dans laquelle
A est l'hydrogène,
A¹ est l'hydrogène ou un groupe méthyle,
Ar est un groupe ortho-phénylène, pyridine-2,3-diyle ou thiophène-2,3-diyle,
E est l'un des groupements suivants : 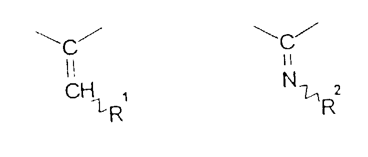 où
R¹ et R² représentent chacun un groupe méthoxy,
G est l'oxygène, un groupe méthylène ou l'un des groupes suivants :
-CH₂-O-, -O-CH₂-, -S(O)ₙ-,-CH₂-S(O)ₙ-, -S(O)ₙ-CH₂-, -C(R⁴)=N-O- O-N=C(R⁴)-, -C(R⁴)=N-O-CH₂-, -N(R⁵)- ou -CH₂-O-N=C(R⁴)-
où
n représente les nombres 0, 1 ou 2,
R⁴ est l'hydrogène, le groupe méthyle ou éthyle et
R⁵ est l'hydrogène, le groupe méthyle ou éthyle, et
Z est un groupe phényle, pyridinyle, pyrimidinyle, pyridazinyle, pyrazinyle, 1,2,3-triazinyle, 1,2,4-triazinyle ou 1,3,5-triazinyle dont chacun est éventuellement substitué, les substituants possibles étant choisis de préférence parmi les substituants suivants :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle, trifluorométhylsulfonyle, méthoxycarbonyle, éthoxycarbonyle, méthoximinométhyle, éthoximinométhyle, méthoximinoéthyle, éthoximinoéthyle ; méthylènedioxy ou éthylènedioxy portant chacun le cas échéant un ou plusieurs substituants, identiques ou différents, fluoro, chloro, méthyle ou éthyle ; ainsi que phényle, phénoxy, benzyle ou benzyloxy portant chacun le cas échéant dans la partie phényle un ou plusieurs substituants, identiques ou différents, fluoro, chloro, bromo, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy.

5. Composition pesticide, caractérisée par une teneur en au moins un composé de formule (I) suivant la revendication 1.

6. Procédé pour combattre des parasites, caractérisé en ce qu'on fait agir des composés de formule (I) suivant la revendication 1 sur des parasites et/ou sur leur milieu.

7. Utilisation de composés de formule (I) suivant les revendications 1 à 4 pour combattre des parasites.

8. Procédé de préparation de compositions pesticides, caractérisé en ce qu'on mélange des composés de formule (I) suivant les revendications 1 à 4 avec des diluants et/ou des agents tensio-actifs.

9. Procédé de production de dérivés d'acide hydroxamique de formule générale (I) suivant la revendication 1, caractérisé en ce qu'on fait réagir des dérivés d'acides carboxyliques de formule générale (II) 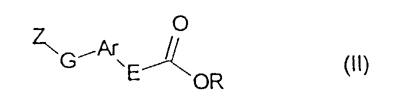 dans laquelle
Ar, E, G et Z ont la définition indiquée dans la revendication 1 et
R est l'hydrogène ou un groupe alkyle,
avec une hydroxylamine de formule générale (III)
A¹-NH-O-A (III)
dans laquelle
A et A¹ ont la définition indiquée dans la revendication 1,
- ou avec un halogénhydrate de ce composé -
le cas échéant en présence d'un accepteur d'acide et en la présence éventuelle d'un diluant.
